# EUROPÄISCHE PATENTSCHRIFT

(11) **EP 3 188 270 B1**
(45) Veröffentlichungstag und Bekanntmachung des Hinweises auf die Patenterteilung: **26.12.2018**
(21) Anmeldenummer: 16181347.2
(22) Anmeldetag: 26.07.2016
(51) Int. Cl.: H01L 51/46

(54) **ORGANISCHES HALBLEITENDES MATERIAL UND DESSEN VERWENDUNG IN ORGANISCHEN BAUELEMENTEN**
ORGANIC SEMI-CONDUCTING MATERIAL AND ITS USE IN ORGANIC COMPONENTS
MATERIAU SEMI-CONDUCTEUR ORGANIQUE ET SON UTILISATION DANS DES DISPOSITIFS ORGANIQUES

(30) Priorität: 30.12.2015 DE 102015123006
(43) Veröffentlichungstag der Anmeldung: 05.07.2017
(73) Patentinhaber: Heliatek GmbH, 01139 Dresden (DE)
(72) Erfinder: Fitzner, Roland, 89077 Ulm (DE); Gerdes, Olga, 89077 Ulm (DE); Hildebrandt, Dirk, 89077 Ulm (DE); D'Souza, Daniel, 01139 Dresden (DE); Mattersteig, Gunter, 89077 Ulm (DE); Weiß, Andre, 01139 Dresden (DE)

(56) Entgegenhaltungen:
- US-A1- 2013 167 930
- OGURA K ET AL: "Greenish metal-lustrous organic crystals formed from 1-aryl-2-(2-thienyl)-5-(5-tricyanoethenyl- 2-furyl)pyrroles", TETRAHEDRON, ELSEVIER SCIENCE PUBLISHERS, AMSTERDAM, NL, Bd. 62, Nr. 10, 6. März 2006 (2006-03-06), Seiten 2413-2419, XP025001640, ISSN: 0040-4020, DOI: 10.1016/J.TET.2005.11.074 [gefunden am 2006-03-06]
- HAI FU ET AL: "Palladium-Catalysed Direct Heteroarylations of Heteroaromatics Using Esters as Blocking Groups at C2 of Bromofuran and Bromothiophene Derivatives: A One-Step Access to Biheteroaryls", SYNLETT, Bd. 23, Nr. 14, 8. August 2012 (2012-08-08), Seiten 2077-2082, XP055358643, DE ISSN: 0936-5214, DOI: 10.1055/s-0031-1290453

## Beschreibung

### Gebiet der Erfindung

Die Erfindung betrifft ein organisches Material der Formel I und deren Anwendung in halbleitenden Bauelementen.

### Stand der Technik

In der organischen Elektronik werden Verschaltungen aus elektrisch leitfähigen Polymeren oder kleinen organischen Molekülen verwendet. Organisch elektronische Bauelemente können dabei zum Beispiel Displays, Datenspeicher oder Transistoren, insbesondere auch Feldeffekt-Transistoren sein. Diese Bauelemente umfassen auch organisch optoelektronische Bauelemente, beispielsweise organische photoaktive Bauelemente wie Solarzellen und Fotodetektoren, die eine photoaktive Schicht umfassen, in der bei Einfall von Strahlung Ladungsträger, z. B. gebundene Elektronen-Loch-Paare (Exzitonen) erzeugt werden. Weitere optoelektronische Bauelemente sind Licht emittierende elektrolumineszierende Bauelemente, die Licht aussenden, wenn sie von Strom durchflossen werden. Optoelektronische Bauelemente umfassen mindestens zwei Elektroden, wobei eine Elektrode auf einem Substrat aufgebracht ist und die andere als Gegenelektrode fungiert. Zwischen den Elektroden befindet sich mindestens eine photoaktive Schicht, vorzugsweise eine organische photoaktive Schicht. Weitere Schichten, beispielsweise Transportschichten, können zwischen den Elektroden angeordnet sein.

US 2013 167930 A1, auch veröffentlicht als EP 2483267 A1, beschreibt verdampfbare organisches Halbleitermaterial zur Verwendung in optoelektronischen Bauelementen.

Ogura, K. u.a.: Greenish metal-lustros organic crystals formed from 1-aryl-2-(2-thienyl)-5-(5-tricyanoethyl-2-furyl)pyrrols. Thetrahedron. Elesvier Science Publishers, Amsterdam, NL, Bd. 62, Nr. 10, 6. März 2006, Seiten 2413-2419. beschreibt die Herstellung und die einzelne Eigenschaften dieser Materialien.

Fu, H.Y. u.a. Palladium-catalysted Direct Heteroarylations of Heteroaromatics Using Esthers as Blocking Groups at C2 of Bromofuran and Bromothiopene Derivates: A One-Step Access to Biheteroaryls. SYNLETT. Georg Thieme Verlag Stuttgart, New York, Bd. 23, Nr. 14, 8.August 2012, Seiten 2077-2082. beschreibt Palladium katalysierte direkte Aryllierung von Heteroarenen mit Methyl-5-Bromfuroate und mit Ethyl-5-Bromothiophene-2-Carboxylat.

Durch den Einsatz geeigneter neuartiger organischer Materialien können verschiedene neuartige Bauelemente, bereitgestellt werden. Dadurch ist die Entwicklung neuer Anwendungen, die dünn, flexibel, leicht sowie mit farblicher Variabilität und auch kostengünstig sind, gegeben.

### Technische Aufgabe

Es werden nach wie vor organische halbleitenden Materialien gesucht, die bei Anwendung in organischen elektronischen Bauelementen zu einer Verbesserung der Eigenschaften der Bauelemente führen.

### Technische Lösung

Erfindungsgemäß wird diese Aufgabe durch die Verbindungen der allgemeinen Formel I gemäß Patentanspruch 1 gelöst. Weitere vorteilhafte Ausgestaltungen der Verbindungen, sowie vorteilhafte Verwendungen der erfindungsgemäßen Verbindungen und organische elektronische Bauelemente enthaltend diese Verbindungen sind Gegenstand weiterer Patentansprüche. Gegenstand der Erfindung nach Patentanspruch 1 sind Verbindungen der allgemeinen Formel I:

EWG1 -(T1)ₐ-(T2)_{b}-(Z)_{c}-(T3)_{d}-(T4)ₑ-EWG2

- mit den Parametern a, b, d, e jeweils unabhängig voneinander 0 oder 1,
- mit dem Parameter c= 1, 2, 3, 4, oder 5,
- wobei die allgemeine Gruppe Z ein Block aus zwei Gruppen M und N ist, verknüpft als *-M-N-* oder *-N-M-*, wobei * die Anknüpfung an die Gruppen T1 bis T4 oder EWG1 oder EWG2 bezeichnet,
- wobei die Gruppen M jeweils unabhängig voneinander ausgewählt sind aus: 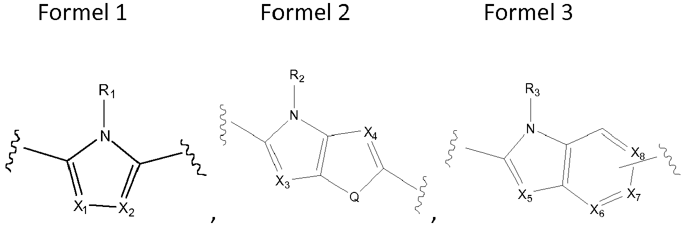
- wobei die Gruppen N jeweils unabhängig voneinander ausgewählt sind aus: 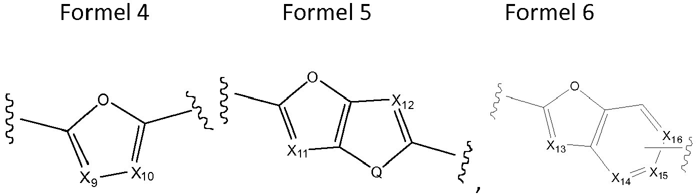
- wobei M und N jeweils derart verknüpft sind, dass mindestens ein N-Atom der Gruppe M und ein O-Atom der Gruppe N jeweils über 2 C-Atome miteinander verbunden sind, und 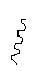 die Anknüpfung an die anderen Gruppen in der Verbindung der allgemeinen Formel I bezeichnet,
- mit X₁-X₁₆ unabhängig voneinander ausgesucht aus N oder C-R, mit der Maßgabe, dass in den Gruppen der Formeln 3 und 6 jeweils eine Gruppe aus den Gruppen X₈/X₇ und X₁₆/X₁₅ die Anknüpfung 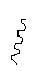 an die anderen Gruppen in der Verbindung der allgemeinen Formel I bezeichnet,
- mit R jeweils unabhängig voneinander ausgesucht aus einer Gruppe aus H, Halogen, verzweigtes oder lineares, zyklisches oder offenkettiges C1-C20 Alkyl, wobei Wasserstoffatome des C₁-C₂₀ Alkyls substituiert sein können, wobei der Substituent insbesondere Halogen, z. B. F sein kann und C-Atome des C₁-C₂₀ Alkyls durch Heteroatome, wie z. B. O oder S ersetzt sein können, C2-C20 Alkenyl, O-Alkyl, S-Alkyl, O-Alkenyl, S-Alkenyl, Alkinyl, Aryl, Heteroaryl, wobei bei allen diesen Gruppen Wasserstoffatome substituiert sein können (substituierte O-Alkyl-Gruppen bevorzugt), CN, NR'R", mit R' und R" jeweils unabhängig voneinander ausgewählt aus: H, verzweigtes oder lineares, zyklisches oder offenkettiges C1-C20 Alkyl, wobei Wasserstoffatome des C₁-C₂₀ Alkyls substituiert sein können, z. B. durch Halogen und C-Atome des C₁-C₂₀ Alkyls durch Heteroatome, z.B. O oder S ersetzt sein können,
- mit R1-R3 jeweils unabhängig voneinander ausgewählt aus einer Gruppe aus H, verzweigtes oder lineares, zyklisches oder offenkettiges C1-C20 Alkyl, wobei Wasserstoffatome des C₁-C₂₀ Alkyls substituiert sein können, bevorzugt durch Halogen, und C-Atome des C₁-C₂₀ Alkyls durch Heteroatome, z.B. O oder S ersetzt sein können, substituiertes oder unsubstituiertes C2-C20 Alkenyl, substituiertes oder unsubstituiertes Aryl, substituiertes oder unsubstituiertes Heteroaryl, CN,
- mit Qjeweils unabhängig voneinander ausgesucht aus S, O, Se, NR''', wobei R''' definiert ist wie R1 bis R3,
- wobei die elektronenziehenden Gruppen EWG1 und EWG2 unabhängig voneinander elektronenziehende Gruppen mit zumindest einer C=C-Doppelbindung sind,
   - wobei die Gruppen T1, T2, T3 und T4 jeweils unabhängig voneinander ausgewählt sind aus: 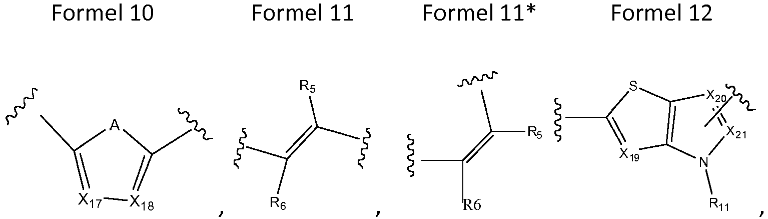 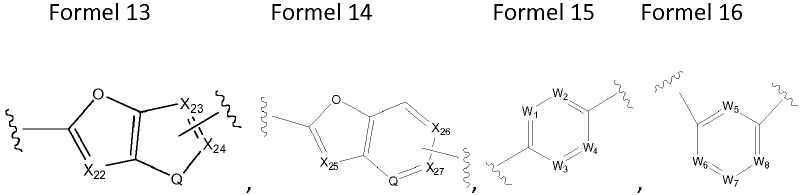
- und 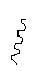 die Anknüpfung an die anderen Gruppen in der Verbindung der allgemeinen Formel I bezeichnet,
- mit R5 und R6 jeweils unabhängig voneinander ausgewählt aus einer Gruppe: H, CN, F, Aryl, Heteroaryl, C2-C20 Alkenyl, C2-C20 Alkinyl, verzweigtes oder lineares, zyklisches oder offenkettiges C1-C20 Alkyl, wobei Wasserstoffatome des C₁-C₂₀ Alkyls substituiert sein können, z. B. durch Halogen, wobei, falls der Substituent R13 in der Verbindung der Formel I vorhanden ist, ein Ringschluss zwischen R5 mit R13 oder R6 mit R13 möglich ist, mit der Maßgabe, dass sich zwischen R5 und R13 oder zwischen R6 und R13 jeweils die Doppelbindung aus Formel 11 oder 11* befindet, möglich ist
- mit W1 bis W8 jeweils unabhängig voneinander ausgewählt aus N, CR, wobei R wie oben beschrieben definiert ist,
- mit X17-X27 unabhängig voneinander ausgesucht aus C-R, wobei R wie oben beschrieben definiert ist und mit der Maßgabe, dass in den Gruppen der Formeln 12, 13 und 14 jeweils eine Gruppe aus den Gruppen X₂₀/X₂₁, X₂₃/X₂₄ und X₂₆/X₂₇ die Anknüpfung 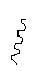 an die anderen Gruppen in der Verbindung der allgemeinen Formel I bezeichnet,
- mit A = S, O, NR"", Se
- mit Q = S, O, NR"", Se
- wobei für die Gruppen A und Q der Substituent R"" jeweils unabhängig voneinander ausgesucht aus H, CN, verzweigtes oder lineares, -zyklisches oder offenkettiges C1-C20 Alkyl, wobei die H-Atome des C1-C20 Alkyl substituiert sein können, wobei insbesondere eine Substitution durch Halogen, z. B. F auftreten kann,

C2-C20-Alkenyl, O-Alkyl, S-Alkyl, O-Alkenyl, S-Alkenyl, Alkinyl, substituiertes oder unsubstituiertes Aryl, substituiertes oder unsubstituiertes Heteroaryl.

Überraschenderweise wurde festgestellt, dass bei den erfindungsgemäßen Verbindungen durch das gemeinsame Strukturelement im Donorblock Z, dass zumindest eine Gruppe M und eine Gruppe N jeweils derart verknüpft sind, dass mindestens ein N-Atom der Gruppe M und ein O-Atom der Gruppe N jeweils über 2 C-Atome miteinander verbunden sind, diese Verbindungen breit und stark Strahlung, insbesondere Licht absorbieren, was in organischen photoaktiven Bauelementen zu erhöhten Effizienzen führen kann. Weiterhin weisen diese Verbindungen auch eine erhöhte Ladungsträgerbeweglichkeit auf, so dass auch organische elektronische Bauelemente, wie z. B. Transistoren, die die erfindungsgemäßen Verbindungen umfassen, verbesserte elektrische Werte aufweisen können. Die erfindungsgemäßen Verbindungen können insbesondere auch als ladungsträgertransportierende Schichten, z. B. p-leitende Materialien verwendet werden. Das Absorptions- und Emissionsspektrum kann sich dabei ausgehend vom unteren UV bis hin zum infraroten Spektralbereich erstrecken. Die oben beschriebene zumindest eine Gruppe M ist dabei ausgewählt aus Pyrrol-Grundkörpern bzw. kondensierten Pyrrol-Gerüsten mit zumindest einem N-Atom der folgenden allgemeinen Formeln 1 bis 3:

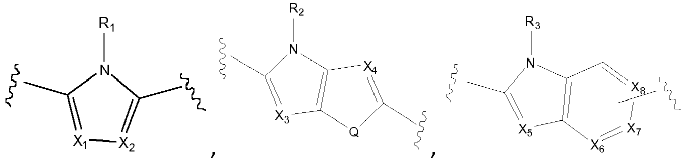

Diese Gruppe M ist direkt mit zumindest einer Gruppe N verbunden, die ausgewählt ist aus Furan-Grundkörpern bzw. kondensierten Furan-Gerüsten mit zumindest einem O-Atom der folgenden allgemeinen Formeln 3 bis 5:

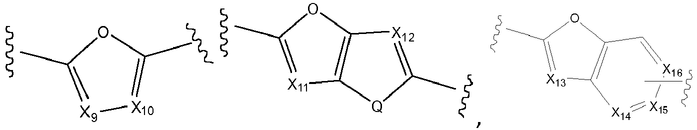

Möglich ist dabei, dass in der Mittelgruppe Z auch mehrere Zweiergruppen aus M-N bzw. N-M Blöcken aufeinander abfolgen, wenn der Parameter c > 1 ist, z. B. *-M-N-M-N-M-N-*, *-M-N-N-M-M-N-* oder *-N-M-N-M-N-M-N-M-*.

Aufgrund dieses strukturellen Merkmales (*-M-N-*)_{c} oder (*-N-M-*)_{c} weisen die vorliegenden Verbindungen eine hohe optische Dichte, bevorzugt im sichtbaren Spektralbereich auf, insbesondere eine hohes Integral über die optische Dichte im Absorptionsspektrum im Vergleich zu nicht erfindungsgemäßen Verbindungen, die das oben beschriebene Strukturelement nicht aufweisen. Unter "Integral" wird dabei der Flächeninhalt unterhalb einer Kurve im Absorptionsspektrum verstanden

Die vorliegenden erfindungsgemäßen Verbindungen der allgemeinen Formel I können dabei neben der immer vorhandenen Elektronendonorgruppe Z weitere Elektronendonorgruppen T1, T2, T3 und T4 aufweisen, die eine weitere Ausdehnung des durch Z bereits vorhandenen konjugierten π-Elektronen-System bedingen. Flankiert werden die Elektronendonorgruppen durch terminale Elektronenakzeptorgruppen EWG1 und EWG2.

Die erfindungsgemäßen Verbindungen sind insbesondere sogenannte "kleine Moleküle", wobei darunter nicht-polymere oligomere organische Moleküle mit einer molaren Masse zwischen 100 bis 2000 g/mol verstanden werden, die insbesondere auch monodispers sein können.

Bevorzugt können die elektronenziehenden Gruppen EWG1 und EWG2 unabhängig voneinander ausgewählt sein aus: 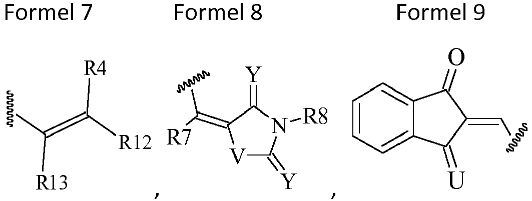
und 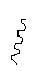 die Anknüpfung an die Gruppen T1 bis T4 oder Z in der Verbindung der allgemeinen Formel I bezeichnet,
mit R4 und R12 jeweils unabhängig voneinander ausgewählt aus H, CN, COOR, mit der Maßgabe, dass R4 und R12 nicht beide H sein können,
wobei R ausgewählt ist aus der gleichen Gruppe von Verbindungen wie bei R1 bis R3 definiert,
mit R13 jeweils unabhängig voneinander ausgewählt aus einer Gruppe: H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, wobei, falls der Substituent R5 oder R6 in der Verbindung der Formel I vorhanden ist, ein Ringschluss zwischen R5 mit R13 oder R6 mit R13 möglich ist, mit der Maßgabe, dass sich zwischen R5 und R13 oder zwischen R6 und R13 jeweils die Doppelbindung aus Formel 11 oder Formel 11* befindet
mit V= O, S
mit Y= O, S, C(CN)₂
mit U = O, S, C(CN)₂
mit R7 und R8 jeweils unabhängig voneinander ausgewählt aus einer Gruppe H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, wobei für jede der Gruppen EWG1 und EWG2 jeweils für jede C=C-Doppelbindung jeweils unabhängig voneinander sowohl das E-Isomer wie auch das Z-Isomer vorliegen kann.

Für jede C=C-Doppelbindung der Formeln 7, 8 und 9 kann also sowohl das E-Isomer ("E" = entgegen; d.h. trans-Konfiguration) wie auch das Z-Isomer ("Z" = zusammen; d.h. cis-Konfiguration) vorliegen, wobei diese Isomere durch eine gedachte Drehung um 180° um die C=C-Doppelbindungsachse gebildet werden. Dies soll im Folgenden anhand des Beispiels des Restes der Formel 8:

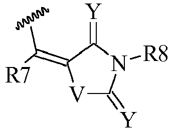

Erläutert werden. Beide Isomere, die voneinander getrennt vorhanden sein können, können durch eine gedankliche Drehung um die C=C-Doppelbindung ineinander überführt werden (angedeutet durch den Pfeil an der Doppelbindung), so dass folgende zwei Isomere für die Gruppe der Formel 8 resultieren:

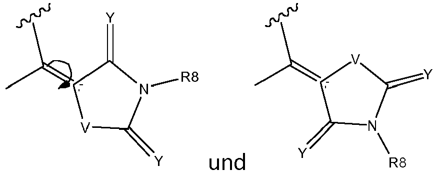

Weiterhin kann insbesondere EWG1 gleich EWG2 sein.

Bei den erfindungsgemäßen Verbindungen der allgemeinen Formel I können die ArylGruppen und die Heteroaryl- Gruppen bevorzugt C5-C10-Aryl und C5-C10-Heteroaryl-Gruppen sein. Als Substituenten werden alle Atome und Atomgruppen außer Wasserstoff verstanden. Als Substituenten kommen insbesondere Halogen, z. B. Fluor aber auch C1-C5-Alkylgruppen, die wiederum substituiert sein können, in Betracht. Bei den O-Alkyl, S-Alkyl, O-Alkenyl, S-Alkenyl, Alkinyl-Gruppen kann es sich jeweils um C1-C20-Gruppen handeln, bevorzugt um C1-C5-Gruppen.

Die zyklischen oder offenkettigen C1-C20 Alkyl-Gruppen der erfindungsgemäßen Verbindungen der Formel I können dabei linear oder auch verzweigt sein und sind bevorzugt C1-C5-Alkyl-Gruppen. Nicht benachbarte und nicht endständige C-Atome dieser Alkyl-Gruppen können durch Heteroatome ersetzt sein.

Unter "Heteroatomen" im Sinne der vorliegenden Verbindungen der Formel I werden insbesondere O, S, Se oder NR""' verstanden, wobei der Substituent R""' definiert ist, wie die Substituenten R₁ bis R₃, die bereits oben beschrieben wurden.

Die Anbindungsstellen bei den einzelnen Gruppen, die mit 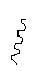 bezeichnet sind kennzeichnen die Anknüpfungspunkte der jeweiligen Gruppen an die anderen Gruppen der Verbindungen der Formel I, also z. B. bei der elektronenziehenden Gruppe EWG1 in der Verbindung der Formel I die Anknüpfung entweder an die Donorgruppen T1 (bei a =1), oder T2 (bei a= 0 und b =1), oder an die Donorgruppe Z wenn die Parameter a und b beide 0 sind.

Die organischen Materialien werden dabei in Form dünner Filme oder kleiner Volumen auf die Folien aufgedruckt, aufgeklebt, gecoated, aufgedampft oder anderweitig angebracht. Für die Herstellung der dünnen Schichten kommen ebenso alle Verfahren in Betracht, die auch für Elektronik auf keramischen oder halbleitenden Trägern verwendet werden.

Gemäß einer weiteren Ausführungsform der vorliegenden Erfindung ist in den Verbindungen der Formel I c = 1 mit der allgemeinen Formel

EWG1 -(T1)ₐ-(T2)_{b}-Z -(T3)_{d}-(T4)ₑ-EWG2

Die Erfinder haben festgestellt, dass ein Donorblock Z ausreichend ist, um eine erhöhte optische Dichte gegenüber strukturell verschiedenen Verbindungen zu erzielen

Weiterhin können die elektronenziehenden Gruppen EWG1 und EWG2 unabhängig voneinander die folgenden Gruppe der Formel 7 sein:

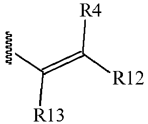

Derartige elektronenziehende Gruppen EWG1 und EWG2 führen zu oligomeren Verbindungen der Formel I, die besonders gut durch Gasphasenabscheidung auf Substrate aufgebracht werden können. Besonders bevorzugt sind R₄ und R₁₂ CN, so dass die besonders stark elektronenziehende Gruppe Dicyano-Vinylen resultiert. Weiterhin kann der Substituent R13 bevorzugt H sein.

Laut einer weiteren Ausführungsform der vorliegenden Erfindung weisen die Verbindungen der Formel I mit c=1 der allgemeinen Formel

EWG1 -(T1)ₐ-(T2)_{b}-Z-(T3)_{d}-(T4)ₑ-EWG2

mit Z = *-M-N-* oder *-N-M-* eine Gruppe M der Formel 1 auf:

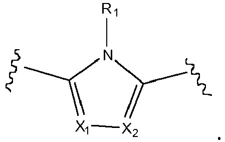

Diese einfachen Pyrrol-Struktureinheiten für den Donorblock M, die kein weiteres kondensiertes aromatisches π-Elektronensystem aufweisen, führen bereits zu einer merklichen Zunahme der Absorption von Strahlung für die erfindungsgemäßen Verbindungen wenn diese weiterhin auch die Donorgruppe N aufweisen. Es ist aber auch möglich, kondensierte Ringsysteme als Donorblock M zu verwenden, die Pyrrol enthalten, wie z. B. Indole oder andere unter die allgemeinen Formeln 2 oder 3 fallende Verbindungen.

Die Begriffe "substituiert" bzw. "Substituent" sind im Sinne der vorliegenden Erfindung so auszulegen, dass ein oder mehrere H-Atome gegen eine beliebige andere Atom-Gruppe oder ein anderes Atom ausgetauscht sind. "Substituenten" in diesem Sinne können insbesondere ein Halogen oder ein Pseudohalogen, z. B. Fluor oder CN, sowie eine Arylgruppe sein, z.B. Phenyl oder eine Alkylgruppe, z. B. eine C1 bis C6-Alkyl-Gruppe.

Die allgemeinen Gruppen und Substituenten in diesem Donorblock der allgemeinen Formel I können dabei folgendermaßen definiert sein: X₁ und X₂ unabhängig voneinander ausgewählt aus C-R mit R jeweils unabhängig voneinander ausgesucht aus einer Gruppe aus H, Halogen, verzweigtes oder lineares, zyklisches oder offenkettiges C1-C20 Alkyl.

Der weitere Donorblock N in Strukturfragment Z kann die folgende allgemeine Gruppe der Formel 4 sein:

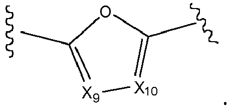

Diese einfachen Furan-Struktureinheiten für den Donorblock N, die kein weiteres kondensiertes aromatisches π-Elektronensystem aufweisen, führen bereits zu einer merklichen Zunahme der Absorption von Strahlung für die erfindungsgemäßen Verbindungen wenn diese weiterhin auch die Donorgruppe M aufweisen. Möglich ist aber auch die Verwendung von kondensierten Donorblöcken, die Furan erhalten, wie z. B. Benzofurane oder anderen unter die allgemeinen Formeln 5 oder 6 fallenden Verbindungen.

Die allgemeinen Gruppen X₉ und X₁₀ in der Formel 4 können dabei bevorzugt unabhängig voneinander ausgewählt sein aus C-R mit R jeweils unabhängig voneinander ausgesucht aus einer Gruppe aus H, Halogen, verzweigtes oder lineares, zyklisches oder offenkettiges C1-C20 Alkyl.

Das konjugierte π-Elektronensystem des Donorbereichs der erfindungsgemäßen Verbindungen der Formel I kann dadurch über den Donorblock Z hinaus erweitert werden, dass zumindest ein weiterer Donorblock T1, T2, T3 oder T4 eingebaut wird und dementsprechend in der Formel I die zu diesen Donorblöcken dazugehörigen Parameter a, b, d oder e sukzessive auf 1 gesetzt werden..

Insbesondere kann a =1 sein und die Gruppe T1 dann bevorzugt ausgewählt sein aus den Gruppen der Formeln 10 und/oder 11:

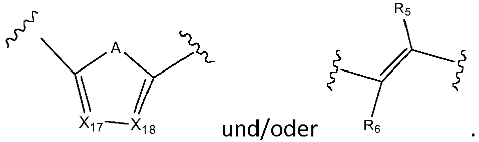

Dabei kann für die Formel 10 insbesondere gelten: A = S oder O.

Weiterhin kann in Formel 10 X₁₇ und X₁₈ = C-R sein, wobei R unabhängig voneinander ausgewählt ist aus einer Gruppe aus H, Halogen, verzweigtes oder lineares, zyklisches oder offenkettiges C1-C20 Alkyl, R5 und R6 jeweils unabhängig voneinander ausgewählt aus H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, wobei falls der Substituent R5 und R6 in der Verbindung vorhanden ist, ein Ringschluss zwischen R5 mit R13 als auch zwischen R6 mit R13 möglich ist, mit der Maßgabe, dass sich zwischen R5 und R13 oder zwischen R6 und R13 die Doppelbindung aus Formel 11 oder Formel 11* befindet.

Falls der Donorblock T2 vorhanden ist, gilt b =1, wobei T2 bevorzugt die allgemeine Gruppe der Formel 10 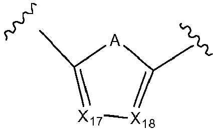 ist.

In der Formel 10 ist bevorzugt A = S, O. Weiterhin kann in der Formel 10 X₁₇ und X₁₈ = C-R sein, wobei R unabhängig voneinander ausgewählt aus einer Gruppe aus H, Halogen, verzweigtes oder lineares, zyklisches oder offenkettiges C1-C20 Alkyl.

Bei Vorhandensein des Donorblocks T3 gilt d =1, wobei die Gruppe T3 bevorzugt ausgewählt ist aus den Gruppen der Formeln 10 oder 11: 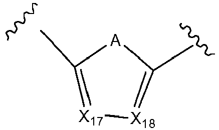 oder 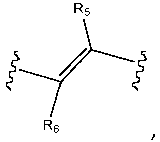 wobei für die Formel 10 gelten kann: A = S oder O.

In der Formel 10 kann insbesondere X₁₇ und X₁₈ = C-R sein, wobei R unabhängig voneinander ausgewählt aus einer Gruppe aus H, Halogen, verzweigtes oder lineares, zyklisches oder offenkettiges C1-C20 Alkyl, R5 und R6 jeweils unabhängig voneinander ausgewählt aus H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, wobei falls der Substituent R5 und R6 in der Verbindung vorhanden ist, ein Ringschluss zwischen R5 mit R13 oder R6 mit R13 möglich ist, mit der Maßgabe, dass sich zwischen R5 und R13 oder zwischen R6 und R13 die Doppelbindung aus Formel 11 oder Formel 11* befindet.

Analog zu T3 kann auch T4 bei e =1 bevorzugt ausgewählt sein aus den Gruppen der Formeln 10 oder 11, wobei die allgemeinen Gruppen und Substituenten für diese Formeln bevorzugt genauso gewählt werden wie bei T3.

Die Erfinder haben festgestellt, dass durch das Vorhandensein insbesondere einer weiteren heterocyclischen Gruppe, die ein Furan- oder ein Thiophenrest sein kann, sowie von Doppelbindungen, die bevorzugt benachbart zu zumindest einer der elektronenziehenden Gruppen EWG1 und/oder EWG2 angeordnet sind, aber auch zwischen einer heterocyclischen Gruppe und dem zentralen Donorblock Z angeordnet sein können, weitere erfindungsgemäße Moleküle hergestellt werden können, welche die bereits genannten vorteilhaften Eigenschaften besitzen.. Weiterhin können die Doppelbindungen (Formel 11 oder Formel 11*) auch benachbart zu beiden elektronenziehenden Gruppen EWG1 und EWG2 vorhanden sein.

Weiterhin ist ein Ringschluss zwischen der Gruppe R5 der Formel 11 oder Formel 11* mit der Gruppe R13 der Formel 7 der elektronenziehenden Gruppen EWG1 und/oder EWG2 oder auch zwischen der Gruppe R6 der Formel 11 oder Formel 11* mit R13 der Formel 7 möglich, mit der Maßgabe, dass sich zwischen R5 und R13 oder zwischen R6 und R13 die Doppelbindung aus Formel 11 befindet, wobei der Ringschluss insbesondere in der Form eines gegebenenfalls substituierten Cyclopentenyl-Rings oder eines gegebenenfalls substituierten Cyclohexenyl-Rings vorhanden ist (siehe z. B. die erfindungsgemäßen Verbindungen Nr. 1 und Nr. 2 in der Tabelle 1).

Gemäß einer weiteren Ausführungsform der vorliegenden Erfindung weisen die erfindungsgemäßen Verbindungen bestimmte bevorzugte Donorblöcke für die Gruppen Z und T1 bis T4 auf, so dass eine allgemeine Strukturformel II resultiert:

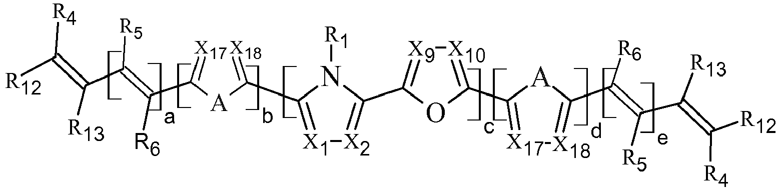

Die Substituenten und allgemeinen Gruppen können dabei so definiert werden, wie bereits beschrieben. Bevorzugt sind dabei folgende Definitionen für die Formel II, wobei aber in allgemeinster Form die Definitionen für die Substituenten und die allgemeinen Gruppen wie für die Verbindungen der Formel I definiert, gelten:
- für A gilt A = O, S.
- unabhängig voneinander gilt für X₁₇ ,X₁₈ , X₁, X₂, X₉ und X₁₀ = C-R, wobei R unabhängig voneinander ausgewählt sind aus einer Gruppe aus H, Halogen, verzweigtes oder lineares, zyklisches oder offenkettiges C1-C20 Alkyl, R5 und R6 jeweils unabhängig voneinander ausgewählt aus H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, und wobei, falls die Substituenten R5 und R6 vorhanden sind, diese einen Ringschluss zwischen R5 mit R13 oder R6 mit R13 bilden können,
- zumindest einer der Parameter a, b, d oder e ist 1, wobei auch alle diese Parameter 1 sein können, bevorzugt ist zumindest einer der Parameter 0, z. B. T4, während alle anderen Parameter 1 sind,
- R4 und R12 sind jeweils unabhängig voneinander ausgewählt sind aus H, CN, mit der Maßgabe, dass R4 und R12 nicht beide H sein können.

Gemäß eines weiteren Aspekts der vorliegenden Erfindung können alle oben beschriebenen erfindungsgemäßen Verbindungen in einem organischen elektronischen Bauelement verwendet werden.

Aufgrund der besonders starken Absorption der erfindungsgemäßen Verbindungen werden besonders gut Exzitonen in Schichten gebildet, die diese Verbindungen umfassen, was bei organischen photoaktiven Bauelementen, die diese Verbindungen umfassen, zu höheren Füllfaktoren FF, verbesserter Leerlaufspannung V_{oc} und verbesserter Kurzschlussstromdichte J_{sc} führt. Bei anderen organischen elektronischen Vorrichtungen sind aufgrund der erhöhten Ladungsträgertransporteigenschaften der erfindungsgemäßen Verbindungen ebenfalls bessere elektronische Werte zu erwarten.

Unter "organischen elektronischen Bauelement" werden dabei alle elektronischen Bauelemente verstanden, die unter Verwendung von organischen leitenden oder halbleitenden Materialien hergestellt werden können, z. B. Transistoren, wie z. B. organische Feldeffekt-Transistoren, organische lichtemittierende Bauelemente, organische photoaktive Vorrichtungen, bei denen in einer photoaktiven Schicht mittels Bestrahlung Exzitonen (Elektron-Loch-Paare) gebildet werden können, wie z. B. Photodetektoren, oder organische Solarzellen.

Die organischen elektronischen Bauelemente weisen dabei in der Regel eine Elektrode und eine Gegenelektrode auf, wobei dazwischen eine organische Funktionsschicht angeordnet ist. Diese organische Funktionsschicht kann dabei eine für die elektronische Funktion des organischen Baudelements wichtige Funktion ausüben, z. B. eine ladungsträgertransportierende Funktion, wie der Transport von Löchern (p-leitend) oder der Transport von Elektronen (n-leitend). Weiterhin kann die organische Funktionsschicht auch eine lichtemittierende Schicht umfassen, die bei Anlegen einer Spannung an die Elektrode und Gegenelektrode durch Rekombination der Löcher (positiven Ladungen) und Elektroden (negative Ladung) Strahlung, z. B. Licht emittiert. Bei der organischen Funktionsschicht kann es sich auch um eine photoaktive Schicht handeln in der bei Bestrahlung mit einer Strahlung, z. B. Licht, oder auch UV-Strahlung oder IR-Strahlung Exzitonen (Elektron-Loch-Paare) gebildet werden. Bei organischen photoaktiven Schichten können insbesondere sogenannte flache Heteroübergänge gebildet werden, bei denen eine flache p-leitende Schicht benachbart zu einer flachen n-leitenden Schicht vorhanden ist und die durch Bestrahlung entweder in der p-leitenden oder n-leitenden Schicht gebildeten Exzitonen an der Grenzfläche zwischen beiden Schichten in Löcher und Elektronen getrennt werden können. Weiterhin kann die photoaktive Schicht auch einen sogenannten Volumenheteroübergang umfassen, bei dem p-leitende und n-leitende Materialien in Form eines interpenetrierenden Netzwerks ineinander übergehen, wobei auch dort die Trennung der durch Bestrahlung gebildeten Exzitonen an den Grenzflächen zwischen p-leitenden und n-leitenden Materialien stattfindet.

Exzitonen sind elektrisch neutrale Anregungszustände, die Elektronen-Loch-Paare, die dann in einem weiteren Schritt an einem p-n Übergang in Elektronen und Löchern getrennt werden. Damit erfolgt die Separation in freie Ladungsträger, die zum elektrischen Stromfluss beitragen. Limitierend ist dabei die Größe der Bandlücke des Halbleiters, entsprechend können nur Photonen absorbiert werden, die eine Energie aufweisen, welche größer als seine Bandlücke ist. Durch das Licht werden immer erst Exzitonen erzeugt, keine freien Ladungsträger, entsprechend ist die rekombinationsarme Diffusion eine für die Höhe des Photostroms wichtige Komponente. Die Exzitonendiffusionslänge muss dabei die typische Eindringtiefe des Lichtes überschreiten, damit ein möglichst großer Teil des Lichtes elektrisch nutzbar ist.

Ein bereits literaturbekannter Aufbau einer gängigen organischen Solarzelle besteht in einer pin- oder nip-Dioden [Martin Pfeiffer, "Controlled doping of organic vacuum deposited dye layers: basics and applications", PhD thesis TU-Dresden, 1999 und WO2011/161108A1]: eine pin Solarzelle besteht dabei aus einem Träger/Substrat mit daran anschließendem meist transparentem Grundkontakt, p-Schicht(en), i-Schicht(en), n-Schicht(en) und einem Deckkontakt. Eine nip-Solarzelle besteht aus einem Träger/Substrat mit daran anschließendem meist transparentem Grundkontakt, n-Schicht(en), i-Schicht(en), p-Schicht(en) und einem Deckkontakt.

Dabei bedeutet n bzw. p-Dotierung, die zu einer Erhöhung der Dichte freier Elektronen bzw. Löcher im thermischen Gleichgewichtszustand führt. Damit sind solche Schichten primär als Transportschichten zu verstehen. Es ist auch möglich, dass n- oder p-Schicht(en) zumindest teilweise nominell undotiert sind und nur aufgrund der Materialeigenschaften (z.B. unterschiedliche Beweglichkeit) oder aufgrund unterschiedlicher Verunreinigungen (z.B. verbliebene Reste aus der Synthese oder der Schichtherstellung) oder durch Einflüsse der Umgebung (z.B. angrenzende Schichten, Eindiffusion von Metallen oder anderen organischen Materialien, Gasdotierung aus der Umgebungsatmosphäre) bevorzugt n-leitende oder p-leitende Eigenschaften besitzen. In diesem Sinne sind derartige Schichten bevorzugt als Transportschichten zu verstehen.

Die Exzitonen gelangen durch Diffusion an eine derartige Grenzfläche, wo Elektronen und Löcher voneinander getrennt werden. Das Material, welches die Elektronen aufnimmt wird als Akzeptor und das Material, welches die Löcher aufnimmt wird als Donor oder Donator bezeichnet.

Die Bezeichnung i-Schicht kennzeichnet eine undotierte oder intrinsiche Schicht. Eine oder mehrere i-Schichten können dabei aus einem Material (planar Heterojunctions) als auch aus einer Mischung zweier oder mehrerer Materialien bestehen, sogenannten bulk heterojunctions, die ein interpenetrierendes Netzwerk aufweisen.

Weiterhin aus der Literatur bekannt sind organische pin-Tandemzellen (DE 102004014046) und pin Mehrfachzellen DE102004014046). Die WO2011/161108A1 offenbart dazu einen Vorschlag zur Realisierung in Form eines photoaktives Bauelements mit einer Elektrode und einer Gegenelektrode, wobei zwischen den Elektroden zumindest ein organisches Schichtsystem angeordnet ist, weiterhin mit mindestens zwei photoaktiven Schichtsystemen und zwischen den photoaktiven Schichtsystemen zumindest zwei verschiedene Transportschichtsysteme des gleichen Ladungsträgertypes, dadurch gekennzeichnet, dass ein Transportschichtsystem energetisch an eines der beiden photoaktiven Schichtsysteme angepasst ist und das andere Transportschichtsystem transparent ausgeführt ist.

Im Folgenden werden Aspekte der vorliegenden Erfindung anhand von Figuren und Ausführungsbeispielen noch näher erläutert.

Es zeigen:
Die Figuren 1 bis 3 die Absorptionsspektren von erfindungsgemäßen Verbindungen im Vergleich zu nicht beanspruchten Verbindungen.

Die Figuren 4 bis 6 Strom-Spannungskurven von organischen photoaktiven Bauelementen (Solarzellen), die erfindungsgemäße Verbindungen enthalten.

Figur 7 ein exemplarisches organisches photoaktives Bauelement im Querschnitt,

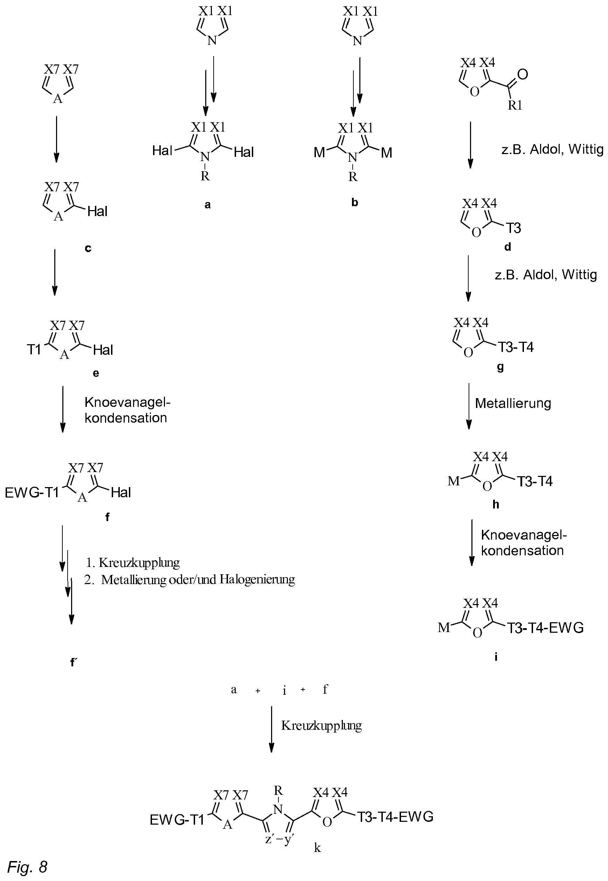

Figur 8 eine Übersicht der Synthese von erfindungsgemäßen Verbindungen.

Tabelle 1 zeigt in einer Übersicht die Strukturen, Schmelzpunkte, und Absorptionsmaxima (in nm und eV im Lösungsmittel (LM)) von Ausführungsbeispielen erfindungsgemäßer Verbindungen, die sowohl unter die allgemeinen Formeln I wie II fallen. Die Synthese dieser Verbindungen wird weiter unten noch im Detail erläutert:

**Tabelle 1:**

| **Nummer** | **Struktur** | **Smp./°C^{a}** | **λmax (LM)/nm^{b}** | **λmax (LM)/eV^{b}** |
|---|---|---|---|---|
| **1** | 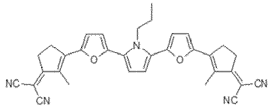 | 290 | 528 | 2,35 |
| **2** | 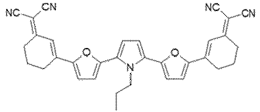 | 292 | 544 | 2,28 |
| **3** | 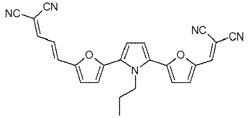 | 224 | 555 | 2,23 |
| **4** | 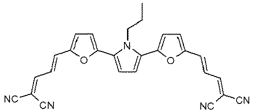 | 261 | 590 | 2,10 |
| **5** | 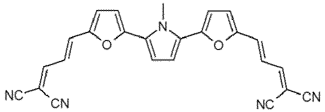 | 231 | 575 | 2,16 |
| **6** | 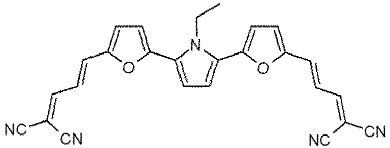 | 263 | 596 | 2,08 |
| **7** | 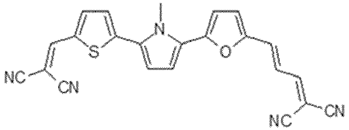 | 265 | 543 | 2,28 |
| **8** | 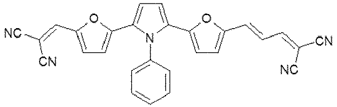 | 254 | 566 | 2,19 |
| **9** | 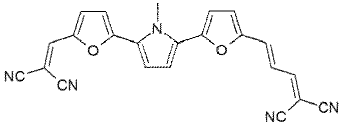 | 246 | 553 | 2,24 |
| **10** | 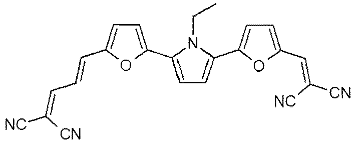 | 245 | 568 | 2,18 |
| **11** | 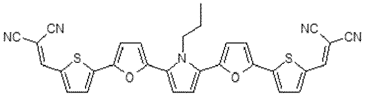 | 243 | 542 | 2,28 |
| 12 | 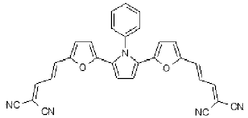 | 279 | 596 | 2,09 |
| 13 | 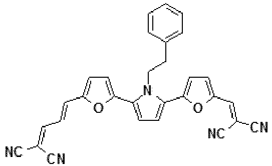 | 206 | 550 | 2,26 |
| 14 | 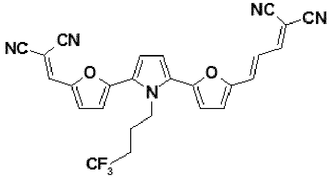 | 212 | 547 | 2,27 |
| 15 | 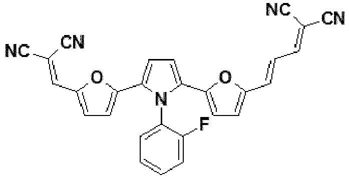 | 255 | 543 | 2,29 |
| 16 | 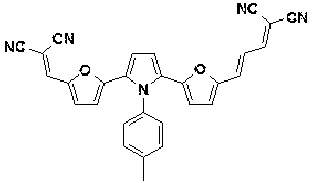 | 292 | 568 | 2,19 |
| **17** | 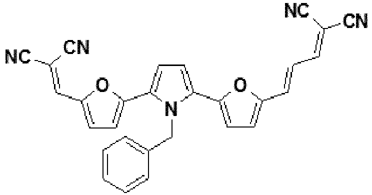 | 264 | 543 | 2,29 |
| **18** | 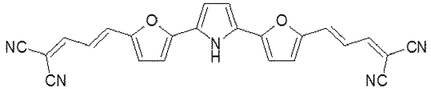 | n.best. | n.best. | n.best. |
| **19** | 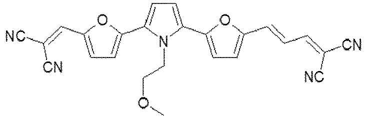 | n.best. | 543 | 2,29 |
| **20** | 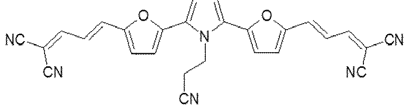 | n.best. | n.best. | n.best. |
| **21** | 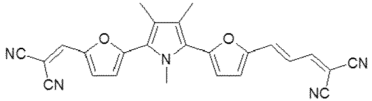 | n.best. | n.best. | n.best. |

| | | | | |
|---|---|---|---|---|
| ^{a} onset DSC (dynamische Differenzkalorimetrie; Beginn des Schmelzbereiches; Extrapolierte Anfangstemperatur (Schnittpunkt Wendetangente-Basislinie) ^{b} in Dichlormethan wenn nicht anders vermerkt | | | | |

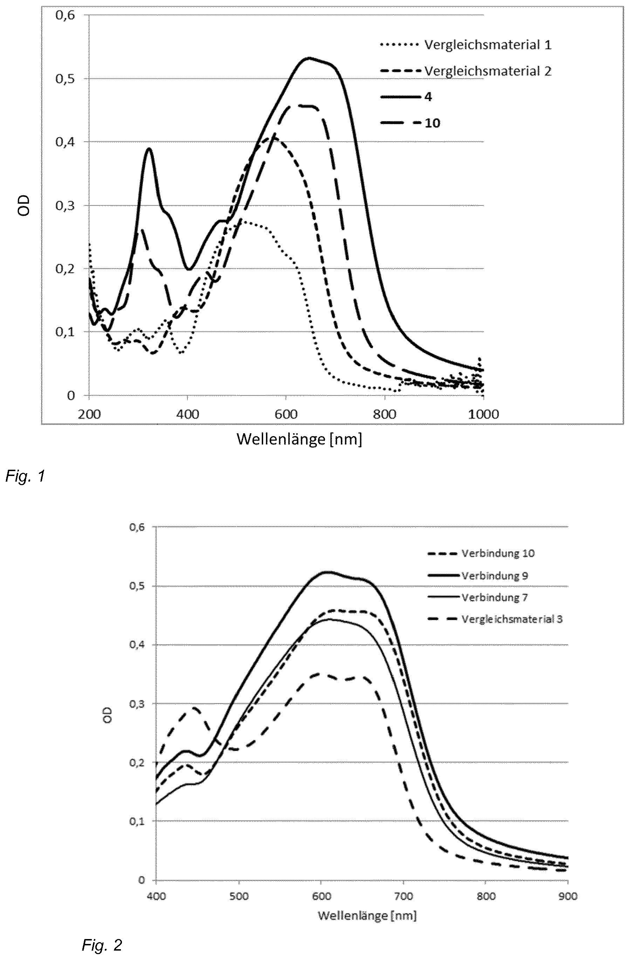

Überraschenderweise wurde gefunden, dass die erfindungsgemäßen Verbindungen eine besonders starke Absorption (d.h. hohe optische Dichte am Absorptionsmaximum oder hohes Integral über die optische Dichte im sichtbaren Spektralbereich im Vergleich zu ähnlichen Verbindungen außerhalb des hier beanspruchten Bereichs) aufweisen. In diesem Zusammenhang zeigt Figur 1 einen Vergleich der Absorptionsspektren (optische Dichte über Wellenlänge in nm) für 30nm dicke vakuumgedampfte Filme für die erfindungsgemäßen Verbindungen 4 und 10 der Tabelle 1 im Vergleich zu zwei nicht erfindungsgemäßen Verbindungen Vergleichsmaterial 1 und Vergleichsmaterial 2.

Die Strukturen und wissenschaftlichen Veröffentlichungen betreffend die Synthesen beider Vergleichsmaterialien sind wie folgt:
Vergleichsmaterial 1: 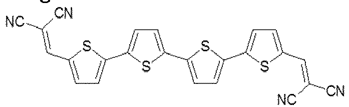 (Fitzner et al., Adv. Funct. Mat. 2011, 21, 897-910)
Vergleichsmaterial **2:** 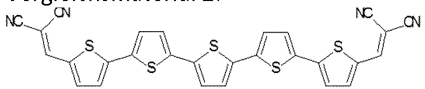 (Fitzner et al., Adv. Funct. Mat. 2011, 21, 897-910).

Den Absorptionsspektren der Figur 1 lässt sich entnehmen, dass die erfindungsgemäßen Verbindungen wesentlich stärker Strahlung absorbieren als die Vergleichsverbindungen und somit ein höheres optisches Integral über die optische Dichte im sichtbaren Spektralbereich aufweisen

Der vorteilhafte synergistische Effekt von erfindungsgemäßen Verbindungen, welche EWG1 und EWG2 -Gruppen in Wechselwirkung mit einem Donorblock, welche eine Furaneinheit direkt neben einer Pyrroleinheit umfassen, zeigt sich auch in folgendem sehr direkten Vergleich der erfindungsgemäßen Verbindungen 7, 9 und 10 der Tabelle 1,
mit der Vergleichsverbindung 3 der folgenden Struktur: 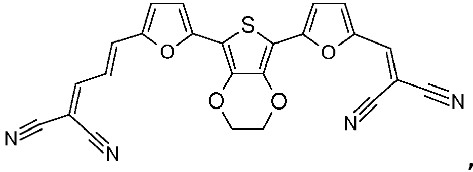 welche statt Pyrrol eine EDOT-Gruppe enthält.

Die folgende Tabelle 2 zeigt verschiedene Parameter dieser Serie von Materialien im direkten Vergleich. Die photovoltaischen Parameter V_{oc}, J_{sc} und FF beziehen sich jeweils auf Solarzellen mit 30nm dicker Mischschicht aus dem jeweiligen Donormaterial dieser Verbindungen und Fulleren C60 als photoaktiver Schicht auf Glas mit dem Aufbau ITO/C60(15 nm)/ den jeweiligen Verbindungen:C60 (30nm)/BPAPF (10nm)/BPAPF:NDP9 (30nm) /NDP9 (1nm)/ Au (50nm), gemessen unter AM1.5 Beleuchtung (Am = Air Mass AM = 1,5 bei diesem Spektrum beträgt die globale Strahlungsleistung 1000 W/m²; AM = 1,5 als Standardwert für die Vermessung von Solarmodulen). ITO dient dabei als Anode, und das benachbarte Fulleren C60 als Elektronentransportschicht ETL, daran schließt sich die photoaktive Schicht als Volumenheteroübergang von C60 als Elektronenakzeptormaterial und der jeweiligen Verbindung als Lochakzeptormaterial (Donatormaterial) an, gefolgt von BPAPF (9,9-bis[4-(N,N-bis-biphenyl-4-yl-amino)phenyl]-9H-fluoren) als Lochtransportschicht HTL und mit NDP9 (Novaled AG) dotiertem BPAPF gefolgt von einer Au-Kathode.

Die spektralen Daten der Figur 2 zeigen die Absorptionsspektren des Vergleichsmaterials 3 im Vergleich mit den erfindungsgemäßen Verbindungen 7, 9 und 10. Die Daten beziehen sich auf 30nm dicke Vakuumaufdampfschichten auf Quarzglas und zeigen, dass die erfindungsgemäßen Verbindungen ein höheres optisches Integral über die optische Dichte im sichtbaren Spektralbereich aufweisen als nicht erfindungsgemäße Verbindungen mit ähnlicher Struktur.

Tabelle 2 zeigt die optische Dichte am Absorptionsmaximum (ODmax), das optische Integral im sichtbaren Bereich (OD-Integral), sowie V_{oc}, J_{sc}, FF und die Effizienz:

| **Substanz** | **ODmax** | **OD-Integral (400-900nm) [nm]** | **V_{oc} [V]** | **J_{sc} [mA/cm²]** | **FF [%]** | **eff [%]** |
|---|---|---|---|---|---|---|
| Verbindung 10 (Werte in Klammern für Zelle mit optimal angepasstem Löchertransport-Material) | 0.45 | 111 / 237 | 0.91 (0.91) | 14.6 (14.9) | 70.8 (73.4) | 9.4 (10.0) |
| Verbindung 9 | 0.53 | 130 / 242 | 0.91 | 13.2 | 71.3 | 8.6 |
| Verbindung 7 | 0.44 | 105 / 232 | 0.94 | 11.7 | 59.3 | 6.5 |
| Vergleichsverbindung 3 | 0.35 | 88 / 307 | 0.91 | 11.5 | 59.1 | 6.2 |

Unabhängig vom Substituenten zeigt Verbindung 10 sowohl höhere Absorptionsmaxima, als auch eine deutlich größere integrale Absorption im sichtbaren Bereich, obwohl die Donorstärken von Pyrrol und EDOT sehr ähnlich sind. Die überlegenen Eigenschaften der erfindungsgemäßen Substanzen (Verbindung 9 und 10) im Vergleich zu Vergleichsmaterial 3 zeigen sich bei identischem Solarzellenaufbau auch in den photovoltaischen Parametern Füllfaktor (70%-73% für Verbindungen 9 und 10 versus 59% bei Vergleichsmaterial 3 bei jeweils 30nm dicken photoaktiven Schichten) und Photostrom J_{sc} (13.2 -14.9mA/cm² für Verbindungen 9 und 10 versus 11.5mA/cm² für Vergleichsverbindung 3). Der deutlich erhöhte FF weist darauf hin, dass die Verbindungen 9 und 10 nicht nur verbesserte Absorptionseigenschaften, sondern auch überlegene Ladungsträgertransporteigenschaften aufweisen. Ein eindrucksvoller Beweis für die außergewöhnlichen Transporteigenschaften der erfindungsgemäß beanspruchten Substanzklasse ist der sehr hohe Füllfaktor von 73%, der für Verbindung 10 gefunden wurde, obwohl Verbindung 10 ein sehr kurzes Oligomer ist, welches eine Doppelbindung weniger als Vergleichsmaterial 1 bzw. drei Doppelbindungen weniger als Vergleichsmaterial 2 umfasst.

Selbst die erfindungsgemäße Verbindung 7 ist noch tendenziell gegenüber der Vergleichsverbindung 3 im Vorteil, obwohl sie durch die sterische Hinderung bei direkter Nachbarschaft zwischen Thiophen und Pyrrol gegenüber den optimierten Verbindungen 9 und 10 (mit Furan auf beiden Seiten des Pyrrols) deutlich abfällt.

In ähnlicher Weise zeigen sich die Vorteile der erfindungsgemäßen Substanzen in einer weiteren Serie direkt vergleichbarer, hier spiegelsymmetrischer Materialien, die jeweils gewisse Strukturelemente mit der erfindungsgemäßen Verbindung 4 der Tabelle 1 gemeinsam haben:
Vergleichsmaterial 4: 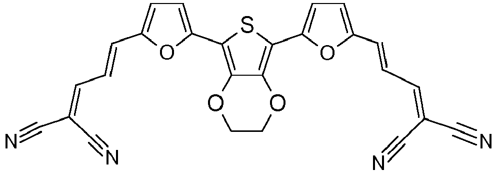
Vergleichsmaterial 5: 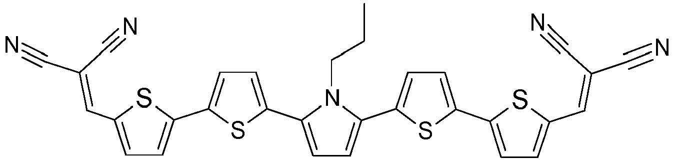
Vergleichsmaterial 6:

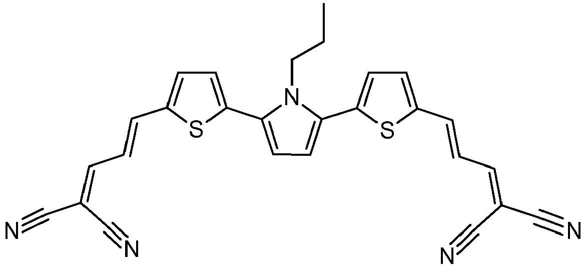

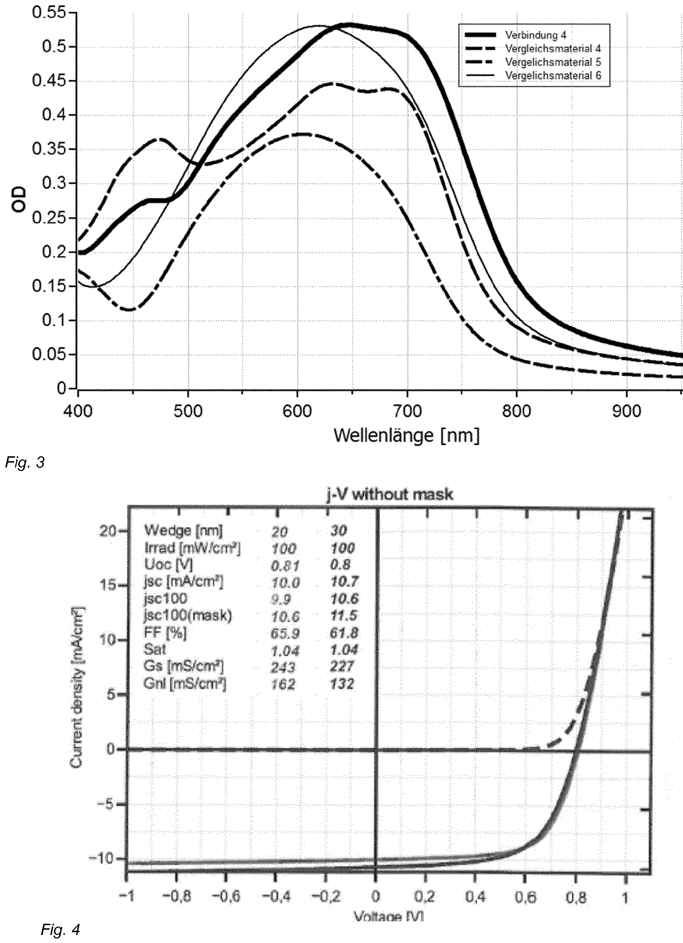

Die Absorptionsspektren der Materialien sind in der Figur 3 gezeigt. Die spektralen Daten beziehen sich auf 30nm dicke Vakuumaufdampfschichten auf Quarzglas.

Die folgende Tabelle 3 zeigt verschiedene Parameter dieser Serie von Materialien im direkten Vergleich. Die photovoltaischen Parameter V_{oc}, J_{sc} und FF beziehen sich jeweils auf Solarzellen mit 30nm dicker Mischschicht aus dem jeweiligen Donormaterial und Fulleren C60 als photoaktive Schicht auf Glas mit dem Aufbau ITO/C60(15 nm) entsprechende Verbindung:C60 (30nm)/HTM81 (10nm)/HTM81:NDP9 (30nm) /NDP9 (1nm)/ Au (50nm), gemessen unter AM1.5 Beleuchtung. Die spektralen Daten beziehen sich auf 30nm dicke Vakuumaufdampfschichten auf Quarzglas.

**Tabelle 3:**

| **Substanz** | **ODmax** | **OD-Integral (400- 900nm) [nm]** | **Voc [V]** | **Jsc [mA/cm²]** | **FF [%]** | **eff [%]** |
|---|---|---|---|---|---|---|
| Verbindung 4 | 0.53 | 156 | 0.81 | 13.3 | 67.4 | 7.3 |
| Verbindung 6 | 0.4* | 114* | 0.81 | 15.1 | 69.7 | 8.5 |
| Vergleichsmaterial 4 | 0.45 | 134 | 0.82 | 12.3 | 66.5 | 6.7 |
| Vergleichsmaterial 5 | 0.37 | 91 | 0.88 | 10.4 | 57.4 | 5.3 |
| Vergleichsmaterial 6 | 0.52 | 127 | 0.9 | 10.4 | 43.1 | 4.0 |

| | | | | | | |
|---|---|---|---|---|---|---|
| * Verbindung 6 zeigt starke Kristallisationsneigung, wodurch die starke Absorption in reinen Schichten auf Glas verschleiert wird. Mischschichten mit Fulleren C60 sind weniger rauh und zeigen die erwartet starke Absorption, was sich in dem außergewöhnlich hohen Jsc-Wert für die Solarzelle auf Mischschichtbasis zeigt. | | | | | | |

Zunächst zeigt die Tabelle 3, dass keine der Vergleichssubstanzen ein ähnlich hohes Absorptionsintegral aufweisen wie Verbindung 4. Am nächsten kommt in dieser Beziehung die strukturell eng verwandte Vergleichsverbindung 6; letztere hat aber ein deutlich schmaleres und weniger strukturiertes Spektrum, was vermutlich auf eine geringere Tendenz zur Selbstorganisation, d.h. weniger geordnete Schichten zurückzuführen ist (sterische Hinderung zwischen Pyrrol und Thiophen), was sich drastisch in dem sehr viel geringeren Füllfaktor der Solarzelle zeigt. Dies gilt in ähnlicher Weise auch für Vergleichsverbindung 5, welche aber sehr viel schwächer absorbiert. Auch Vergleichsmaterial 4 mit EDOT an Stelle der erfindungsgemäßen Pyrrol-Einheit fällt - ähnlich wie oben gezeigt Vergleichsmaterial 3 in Bezug auf Verbindung 10 - gegenüber Verbindung 4 in wesentlichen Parametern deutlich ab.

Es konnte darüber hinaus gezeigt werden, dass viele Derivate der erfindungsgemäßen Verbindungen nicht nur Licht absorbieren sondern auch rückstandsfrei im Vakuum verdampft werden können, während z.B. die oben gezeigte Vergleichsverbindung 6 (mit Thiophen statt Furan wie in Verbindung 4) einen großen zersetzten Rückstand in der Verdampferquelle aufweist.

Durch sehr gute Ladungstransporteigenschaften und gute Absorptionseigenschaften (s.oben) können hohe Photoströme mit exzellenten Füllfaktoren erzeugt werden. Damit können sehr gut kombinierte Tandem-/Tripel-/ Quadrupel-/ oder Multijunction-Solarzellen hergestellt werden.

Figur 4 zeigt die Strom-Spannungskurve mit einer BHJ-Zelle mit dem Aufbau: ITO/C60(15 nm) Verbindung 4:C60 (20/30nm, 1:1, 70 °C)/BPAPF (10nm)/BPAPF:NDP9 (30nm, 9,1%wt) /NDP9 (1nm)/ Au (50nm), wobei die photoaktive Schicht ein Volumenheteroübergang (bulk heterojunction BHJ) ist.

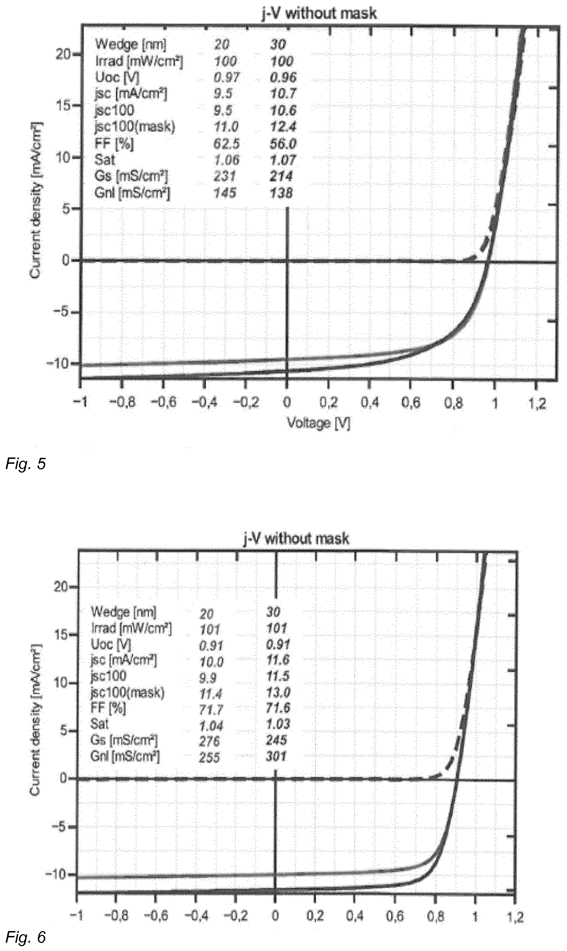

Figur 5 zeigt die Strom-Spannungskurve mit einer BHJ-Zelle mit dem Aufbau: ITO/C60(15 nm) Verbindung 8:C60 (20/30nm, 3:2, 70 °C)/BPAPF (10nm)/BPAPF:NDP9 (30nm, 9,7%wt) /NDP9 (1nm)/ Au (50nm), wobei die photoaktive Schicht ein Volumenheteroübergang (bulk heterojunction BHJ) ist.

Figur 6 stellt die Strom-Spannungskurve mit einer BHJ-Zelle mit dem Aufbau ITO/C60(15 nm) Verbindung 10:C60 (20/30nm, 3:2, 50 °C)/BPAPF (10nm)/BPAPF:NDP9 (30nm, 10,6%wt) /NDP9 (1nm)/ Au (50nm) dar, wobei die photoaktive Schicht ein Volumenheteroübergang (bulk heterojunction BHJ) ist.

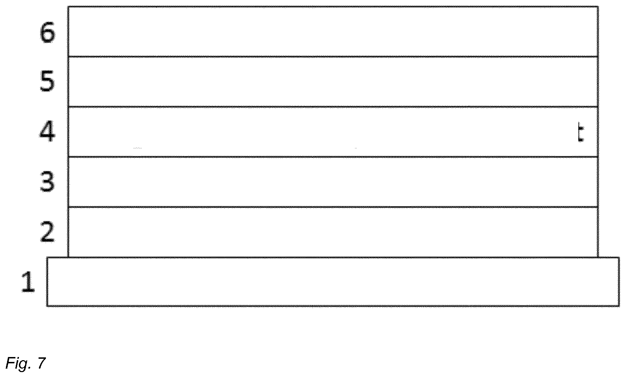

Die Figur 7 zeigt eine beispielhafte photoaktive Vorrichtung mit einem Substrat 1, z. B. aus Glas auf dem sich eine Kathode als Elektrode 2 befindet, die z. B. ITO umfassen kann. Darauf angeordnet sind eine elektronentransportierende Schicht 3 als ETL sowie eine photoaktive Schicht 4, die die erfindungsgemäßen Verbindungen als p-leitende Donorkomponente und weiterhin eine n-leitende Komponente als Elektronenakzeptor-Komponente, z. B. C60 entweder als flacher Heteroübergang oder als Volumenheteroübergang enthalten. Darüber angeordnet befindet sich eine lochleitende Schicht 5 als HTL und die Anode 6. Photoaktive Bauelemente mit den erfindungsgemäßen Verbindungen können weitere funktionelle Schichten umfassen und z. B. auch als Mehrfachzellen oder Tandemzellen konzipiert sein.

Im Folgenden sollen noch die Synthesen der konkreten Ausführungsbeispiele und ein allgemeiner Syntheseweg nach dem Baukastensystem für die erfindungsgemäßen Verbindungen erläutert werden.

### Synthese

Vorteilhafterweise können die erfindungsgemäßen Absorbermoleküle nach einem einfachen Baukastensystem leicht und in guten Ausbeuten zugänglich gemacht werden. Beispielhaft ist im Folgenden die Synthese der erfindungsgemäßen Verbindung der allgemeinen Formel (I) dargestellt.

Die Synthese der allgemeinen Verbindung (I) kann nach einer der im Folgenden beschriebenen Methoden erfolgen. Diese soll hier als beispielhafte Darstellung dienen und kann in der Reihenfolge ihrer einzelnen Schritte variiert, oder durch andere bekannte Methoden abgewandelt werden. Auch die Zusammenfassung einzelner Reaktionsschritte oder die Veränderung von Teilen der Syntheseroute ist möglich.
Der Substituent Hal- steht für Halogenkomponente, bei der es sich typischerweise um ein Halogenatom, aber auch andere in Kreuzkupplungsreaktionen einsetzbare funktionelle Gruppen, wie beispielsweise Carbonsäuren oder Triflate, oder weitere geeignete Gruppen einschließlich -H handeln kann. Der Substituent Met- steht für Metallkomponente, worin im weiteren Sinne metall- oder halbmetallhaltige funktionelle Gruppen, oder andere, auch metall-freie, in Kreuzkupplungsreaktionen verwendbare funktionelle Gruppen, einschließlich -H gemeint sind. Diese Gruppe Met kann insbesondere ausgewählt sein aus einer der folgenden funktionellen Gruppen:

-SnR*3, -B(OR*)2, -Zn-Hal*, -Mg-Hal*,

wobei es sich bei R* um ein C1-C10 Alkyl handelt und wobei es sich bei der Gruppe Hal* um ein Halogen handelt, insbesondere ausgewählt aus der Gruppe enthaltend: Cl, Br, I. Der Baustein Z der allgemeinen Verbindung (I) enthaltend M-N oder N-M kann somit über dem Fachmann bekannte C-C-Kupplungsreaktionen hergestellt werden:

M-Met + N-Hal → N-M

oder

N-Met + M-Hal → N-M

Wobei "Hal" einen Halogensubstituenten bedeutet, insbesondere ausgewählt aus der Gruppe enthaltend: Cl, Br, I. Die hierfür einzusetzenden Ausgangsverbindungen sind entweder kommerziell erhältlich, oder durch typische Metallierungs- oder Halogenierungsreaktionen zugänglich. Die Kopplung zum Baustein N-M beziehungsweise M-N kann beispielsweise durch Suzuki-, Negishi- oder Stille-, Kumada- oder Hiyama- und weitere Kopplungsreaktionen durchgeführt werden, die unter anderem in "Metal-Catalyzed Cross-Coupling Reactions, 2nd, Completely Revised and Enlarged Edition" (Wiley VCH, ISBN: 978-3-527-30518-6) beschrieben sind (Suzuki: Seiten 41-123, Negishi: Seiten 619-670, Stille: Seiten 125-161, Kumada: Seiten 671-698, Hiyama: Seiten 163-216, weitere Kopplungsreaktionen: Seiten 815-889). In der Regel, aber nicht ausschließlich erfolgen die C-C-Kreuzkupplungsreaktionen unter Verwendung eines Katalysators.
Die Einführung weiterer Gruppen ausgewählt aus N, M oder T1 bis T4 kann wiederum dadurch erfolgen, dass eine der beiden Komponenten metalliert und die zweite Kopplungskomponente halogeniert oder andersartig substituiert wurde, um für C-C-Kopplungsreaktionen geeignet aktiviert zu werden. Dabei ist grundsätzlich variabel, welche Kopplungskomponente mit welcher aktivierenden Gruppe ausgestattet wird. Üblicherweise werden in C-C-Kopplungsreaktionen dann hohe Reaktionsausbeuten erzielt, wenn der elektronenreichere Baustein den "Met-Substituenten" und der elektronenärmere Baustein den "Hal-Substituenten" trägt. Aber auch die inverse Reaktionsführung kann zu guten Ergebnissen führen. Die Kopplung der weiteren Bausteine kann dann wiederum durch dem Fachmann bekannte Kopplungsrektionen, wie beispielsweise Suzuki-, Negishi- oder Stille-, Kumada- oder Hiyama-Kopplungsreaktionen durchgeführt werden. Die Auswahl einer geeigneten Kopplungsreaktion trifft der Fachmann in Hinblick auf die erforderlichen Reaktionsbedingungen und deren Verträglichkeit mit etwaig vorhanden funktionellen Gruppen. Dabei können, je nach Ausführung der Zielverbindung, ein oder mehrere Bausteine pro Reaktionsschritt an das Gerüst gekoppelt werden.

N-M → N-M-Hal → N-M-T

N-M → Hal-N-M → T-N-M

N-M → N-M-Met → N-M-T

N-M → Met-N-M → T-N-M

N-M → Met-N-M-Met → T-N-M-T

N-M → Hal-N-M-Hal → T-N-M-T

Handelt es sich bei einem Baustein T um eine Komponente der Formel 11 oder Formel 11*, so kann dieser Baustein nach einer üblichen, dem Fachmann bekannten Route zur Einführung von Doppelbindungen geschehen. Dies können beispielsweise Heck-, Wittig-, Aldolreaktionen sein, oder aber auch Eliminierungen, Cope- oder McMurry-Reaktionen oder die bereits gennannten C-C-Kopplungsreaktionen sein, die unter anderem in March's Advanced Organic Chemistry: Reactions, Mechanisms, and Structure, 7th Edition (ISBN: 978-0-470-46259-1) beschrieben sind (Kapitel 12, S. 649ff, Kapitel 13, S. 732ff, Kapitel 16, S. 1067ff, Kapitel 17, S. 1253ff, Kapitel 19, S. 1433ff.).
Nach den beschriebenen Methoden können beliebige weitere Bausteine aus der Gruppe N, M oder T eingeführt werden.

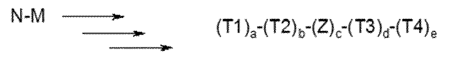

Die Einführung der elektronenziehenden Gruppen EWG1 und EWG2 erfolgt in der Regel durch eine Aldolkondensation einer eine aktivierte Methyleneinheit tragenden Komponente der Formeln 7, 8 oder 9 mit einer Carbonylkomponente, welche vorher an die benachbarte Gruppierung T, M oder N durch dem Fachmann bekannte Methoden wie beispielsweise Gattermann, Gattermann-Koch, Houben-Hoesch, Vilsmeier/ Vilsmeier-Haack, Friedel-Crafts-Acylierung oder nach Lithiierung durch eine Umsetzung mit einem Säurederivat oder Carbonylierungsreagenz eingeführt wird, die unter anderem in Organikum (ISBN 978-3-527-33968-6 - Wiley-VCH, Kapitel D2-D9) beschrieben sind.

(T1)ₐ-(T2)_{b}-(Z)_{c}-(T3)ₐ-(T4)ₑ → carbonyl-(T1)ₐ-(T2)_{b}-(Z)_{c}-(T3)_{d}-(T4)ₑ-carbonyl

carbonyl-(T1)ₐ-(T2)ₐ-(Z)_{c}-(T3)_{d}-(T4)ₑ-carbonyl → EWG-(T1)ₐ-(T2)_{b}-(Z)_{c}-(T3)_{d}-(T4)ₑ-EWG

wobei die Gruppe "carbonyl" die oben genannte Carbonyl-Komponente ist. Die Reihenfolge der beschriebenen Syntheseschritte ist beliebig variierbar. So ist es beispielsweise möglich, zwei Molekülteile der allgemeinen Formel (I) nach einer der oben beschriebenen Methoden aufzubauen und die Bindung zwischen den Komponenten M-N, N-N, M-M, N-T, M-T oder T-T im letzten Reaktionsschritt auszubilden.

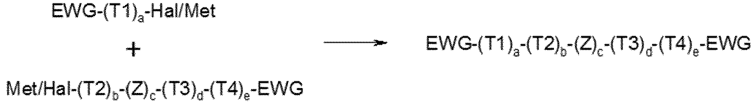

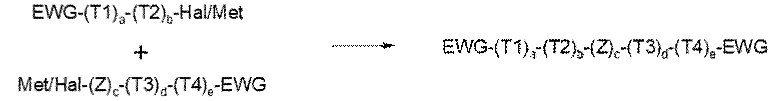

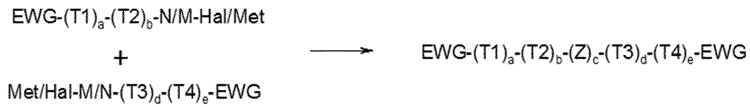

Erfindungsgemäße Verbindungen wurden über im Folgenden dargestellte Methodik synthetisiert, entweder anhand einer
a. doppelten Stille-Kopplung
b. doppelten inversen Stille-Kopplung oder
c. einfachen Stille-Kopplung.

Nachfolgend sind die entsprechenden Allgemeinen Arbeitsvorschriften (AAV1 bis AAV3) für die Versionen a, b und c aufgeführt:

### a. Allgemeine Arbeitsvorschrift (AAV1)

1 mmol Distannylverbindung Edukt 1 und 2,5 mmol Edukt 2 wurden in 4 ml entsprechendem Lösemittel (Tabelle 4) gelöst und die Lösung wurde entgast. Anschließend wurde 0,05 mmol Pd-Katalysator dazu gegeben und das Reaktionsgemisch wurde über Nacht erhitzt. Das Reaktionsgemisch wurde auf Raumtemperatur gebracht, dabei ausgefallener Niederschlag abfiltriert und mit Methanol nachgewaschen. Das Rohprodukt wurde aus entsprechendem Lösemittel umkristallisiert.

### Tabelle 4:

**Tabelle 4: Reaktionsbedingungen zur Synthese der Verbindungen 1,2,4,5,11**

| **Nummer** | **Edukt1** | **Edukt 2** | **Reaktionsbedingungen** | **Ausbeute (%)** | **Umkristallisiert aus** |
|---|---|---|---|---|---|
| **1** | **A1** | **B8** | Pd(PPh₃)₄/DMF/80°C | 32 | Chlorbenzol |
| **2** | **A1** | **B6** | Pd(PPh₃)₄/DMF/80°C | 34 | Chlorbenzol |
| **4** | **A1** | **B2** | Pd(PPh₃)₄/DMF/80°C | 27 | Chlorbenzol |
| **5** | **A2** | **B2** | Pd(PPh₃)₄/1,4-Dioxan/80°C | 34 | Chlorbenzol |
| **11** | **A1** | **C14** | Pd₂(dba)₃/P(*tert-*Bu)₃/1,4-Dioxan/80°C | 43 | Chlorbenzol |

### b. Allgemeine Arbeitsvorschrift (AAV2)

1 mmol **Dibromverbindung** und 2,5 mmol **B4** wurden in 4 ml Dioxan gelöst und die Lösung wurde entgast. Anschließend wurde 0,05 mmol Pd-Katalysator dazu gegeben und das Reaktionsgemisch wurde über Nacht bei 80°C gerührt. Das Reaktionsgemisch wurde auf Raumtemperatur gebracht, dabei ausgefallener Niederschlag abfiltriert und mit Methanol nachgewaschen.

**Tabelle 5: Reaktionsbedingungen zur Synthese von Verbindung 6 und 12**

| **Nummer** | **Edukt1** | **Edukt 2** | **Reaktionsbedingungen** | **Ausbeute (%)** | **Umkristallisiert aus** |
|---|---|---|---|---|---|
| **6** | **A3** | **B4** | Pd₂dba₃ / P(t-Bu)_{3·}HBF₄)/80°C | 40 | Chlorbenzol |
| **12** | **A8** | **B4** | Pd₂dba₃ / P(t-Bu)₃ / Dioxan/60°C | 13 | Tetrahydrofura n/Hexan |

### c. Allgemeine Arbeitsvorschrift (AAV3)

In einem mit Argon inertisierten Schlenkgefäß wurde 1 mmol Halogenverbindung Edukt 1 und 1.2 mmol 2-[3-(5-Trimethylstannanyl-furan-2-yl)-allyliden]-malononitril **B4** Edukt 2 in 3 ml Lösungsmittel gelöst. Die Lösung wurde entgast, anschließend 0.05 mmol Pd-Katalysator zugegeben und das Reaktionsgemisch über Nacht unter Rühren erhitzt. Das Reaktionsgemisch wurde auf Raumtemperatur abgekühlt, der entstandene Niederschlag abfiltriert und mit Methanol nachgewaschen. Das Rohprodukt wurde aus dem jeweiligen Lösungsmittel (Tabelle 5) umkristallisiert.

**Tabelle 6: Reaktionsbedingungen zur Synthese der Verbindungen 3,7,8,9,10, 13-17**

| **Nummer** | **Edukt1** | **Edukt 2** | **Reaktionsbedingungen** | **Ausbeute / %** | **umkristallisiert aus** |
|---|---|---|---|---|---|
| **3** | **C4** | **B4** | Pd2(dba)3 / P(tBu)3, 1,4-Dioxan, 80 °C | 72 | Tetrahydrofuran/ Hexan |
| **7** | **C6** | **B4** | Pd2(dba)3 / P(tBu)3, 1,4-Dioxan, 80 °C | 62 | Tetrahydrofuran |
| **8** | **C10** | **B4** | Pd2(dba)3 / P(tBu)3, 1,4-Dioxan, 80 °C | 26 | Tetrahydrofuran / Hexan |
| **9** | **C2** | **B4** | Pd(PPh₃)₄, Tetrahydrofuran, 65 °C | 9 | Tetrahydrofuran/ Hexan |
| **10** | **C8** | **B4** | Pd2(dba)3 / P(tBu)3, 1,4-Dioxan, 80 °C | 48 | Tetrahydrofuran/ Hexan |
| **13** | **C25** | **B4** | Pd2(dba)3 / P(tBu)3, 1,4-Dioxan, 80 °C | 20 | säulenchromatographisch gereinigt (Dichlormethan) |
| **14** | **C27** | **B4** | Pd(PtBu3)2 1,4-Dioxan, 80 °C | 39 | Toluol |
| **15** | **C37** | **B4** | Pd(PtBu3)2 1,4-Dioxan, 60 °C | 59 | Toluol |
| **16** | **C23** | **B4** | Pd(PtBu3)2, 1,4-Dioxan, 60 °C | 55 | Chlorbenzol |
| **17** | **C39** | **B4** | Pd(PtBu3)2, 1,4-Dioxan, 60 °C | 80 | Tetrahydrofuran/ Hexan |

Alternativ können erfindungsgemäße Verbindungen auch über andere bekannte C-C-Kopplungsreaktionen wie beispielsweise Suzuki- oder Neghishi-Reaktion erfolgen.

Die Synthese der Edukte 1 (A), Edukte 2 (B) und Edukte 3 (C) kann nach folgenden Vorschriften erfolgen:

### Synthese der Verbindungen A1-A19

### N-Propyl-2,5-bis-trimethylstannyl-pyrrol (A1)

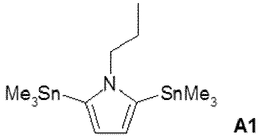

Die Verbindung wurde gemäß der Literatur von G. H. Jana et al. Bioorg. Med. Chem. Lett., 2015, (15), 3592-3595 hergestellt. Anstelle von Tributylzinnchlorid wurde Trimethylzinnchlorid verwendet.
Das Rohprodukt wurde aus Methanol umkristallisiert und man erhielt Produkt **A1** in 35% Ausbeute als farblosen Feststoff. ¹H-NMR (CDCl₃): 6.40 ppm (s, 2H), 3.88 (m, 2H), 1.76 (m, 2H), 0.97 (t, 3H), 0.32 (s, 18H).

### N-Methyl-2,5-bis-trimethylstannyl-pyrrol (A2)

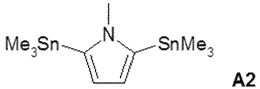

Die Verbindung wurde gemäß der Literatur von G. H. Jana et al. Bioorg. Med. Chem. Lett., 2015, (15), 3592-3595 hergestellt. Anstelle von Tributylzinnchlorid wurde Trimethylzinnchlorid verwendet.
Das Rohprodukt wurde aus *iso-*propanol umkristallisiert und man erhielt Produkt **A2** in 34% Ausbeute als farblosen Feststoff. ¹H-NMR (CDCl₃): 6.39 ppm (s, 2H), 3.75 (s, 3H), 0.32 (s, 18H).

### 2,5-Dibrom-1-ethyl-pyrrol (A3)

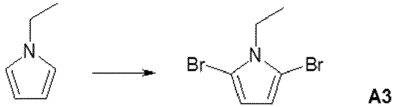

951 mg (10,0 mmol) 1-Ethylpyrrol wurde in 50 ml THF bei -78°C unter Argonatmosphäre gelöst. 3,60 g (20,0 mmol) NBS wurde innerhalb von 15 min hinzugegeben. Es wurde bei - 78°C für 4 h gerührt und anschließend über Nacht auf RT erwärmt. Die Reaktionsmischung wurde mit 100 ml ges. Na₂SO₃-Lösung versetzt und zweimal mit MTBE extrahiert. Die vereinigten organischen Phasen wurden mit ges. NaCl-Lösung gewaschen. Man trocknete über Na₂SO₄, filtrierte und entfernte die Lösemittel im Vakuum. Der Rückstand wurde chromatographisch über Kieselgel gereinigt und man erhielt 1,00 g Produkt **A3** (40%) als farbloses Öl. GC-MS (El, 75eV) *m*/*z* 252.9 (M⁺, 100%).

### 1-Methyl-2-trimethylstannanyl-1H-pyrrol (A4)

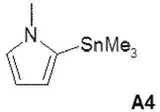

Die Synthese erfolgte analog zu Groenendaal et al. Synth. Commun. 1995, 25 (10), 1589-1600

### 1-Propyl-2-trimethylstannanyl-1H-pyrrol (A5)

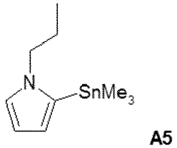

Die Synthese erfolgte analog zu Groenendaal et al. Synth. Commun. 1995, 25 (10), 1589-1600

### 1-Ethyl-2-trimethylstannanyl-1H-pyrrol (A6)

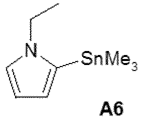

Die Synthese erfolgte analog zu Groenendaal et al. Synth. Commun. 1995, 25 (10), 1589-1600

### 1-Phenyl-2-trimethylstannanyl-1H-pyrrol (A7)

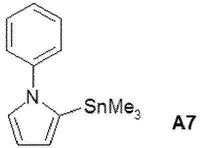

Die Synthese erfolgte analog zu Groenendaal et al. Synth. Commun. 1995, 25 (10), 1589-1600

### 2,5-Dibrom-1-phenyl-pyrrol (A8)

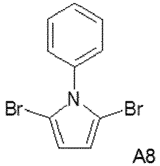

1446 mg (10,0 mmol) 1-Phenylpyrrol wurde in 50 ml THF bei -78°C unter Argonatmosphäre gelöst. 3,60 g (20,0 mmol) NBS wurde innerhalb von 15 min hinzugegeben. Es wurde bei -78°C für 4 h gerührt und anschließend über Nacht auf RT erwärmt. Die Reaktionsmischung wurde mit 100 ml ges. Na₂SO₃-Lösung versetzt und zweimal mit MTBE extrahiert. Die vereinigten organischen Phasen wurden mit ges. NaCl-Lösung gewaschen. Man trocknete über Na₂SO₄, filtrierte und entfernte die Lösemittel im Vakuum. Der Rückstand wurde chromatographisch über Kieselgel gereinigt und man erhielt 2,75 g Produkt **A8** (91%) als farbloser Feststoff. ¹H-NMR (Aceton-d6): 7.57 ppm (m, 3H), 7.31 (dd, 2H), 6.38 (s, 1H).

### Allgemeine Vorschrift zur Synthese von Pyrrolen aus Dimethoxytetrahydrofuran

Die Synthesen erfolgen analog zu Literaturvorschrift von Sunil Kumar et al., J. Phys. Chem. C, 2014, 118 (5), 2570:
50 mmol Natriumacetat wurde in 100 ml entmineralisiertes Wasser bei Raumtemperatur gelöst und 50 mmol entsprechendes Amin zugegeben. 25 ml Eisessig wurden langsam dazu getropft und das Gemisch wurde auf 80°C erwärmt. 50 mmol 2,5-Dimethoxytetrahydrofuran wurden zugetropft und das Reaktionsgemisch wurde für 16 h bei 80°C gerührt. Anschließend wurde die Reaktionslösung auf Raumtemperatur gebracht und mit Dichlormethan extrahiert. Die organische Phase wurde mit ges. NaCl-Lösung gewaschen, über Natriumsulfat getrocknet und am Rotationsverdampfer eingeengt. Das Rohprodukt wurde chromatographisch gereinigt.

| **Struktur** | **Nummer** | **Ausbeute** | **Charakterisierung** |
|---|---|---|---|
| 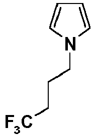 | A13 | 82 | GC-MS m/z: 177 [M] |
| 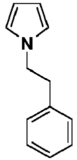 | A9 | 96 | GC-MS m/z: 171 [M], 104, 80 |
| 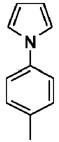 | A16 | 80 | GC-MS m/z: 132 [M] |

### Synthese von 1-Phenethyl-2-trimethylstannyl-1H-pyrrol (A10)

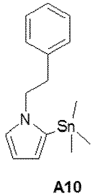

Die Synthese von A10 erfolgte analog zu Groenendaal et al. Synth. Commun. 1995, 25 (10), 1589-1600

### 1-(2-Fluoro-phenyl)-1H-pyrrol (A17)

### 1-Benzyl-1H-pyrrol (A19)

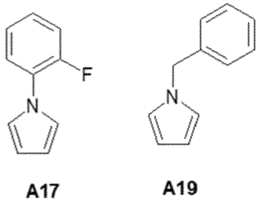

Die Verbindungen A17 und A19 sind kommerziell erhältlich

### Synthese der Verbindungen B1-B8

### (E)-3-(5-Brom-furan-2-yl)-propenal (B1)

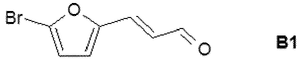

Die Synthese von **B1,** erfolgt gemäß der Literatur von I. I. Popov, Z. N. Nazarova, A. P. Chumak, Chem. Heterocycl. Compd., 1978, 14, (3), 253-255.

50 mmol 5-Brom-2-furfural wurden in 100 ml 6%ige NaOH-Lösung suspendiert. Man tropfte Acetaldehyd in 15 ml Wasser zum Reaktionsgemisch bei 0°C. Es wurde 1h bei 0°C nachgerührt. Der Niederschlag wurde abfiltriert, mit Wasser gewaschen und getrocknet. Das Rohprodukt wurde chromatographisch über Kieselgel gereinigt. Ausbeute 74%. 1H-NMR (Aceton-d6): 9.64 ppm (d, 1H), 7.44 (d, 1H), 7.04 (d, 1H), 6.73 (d, 1H), 6.47 (dd, 1H).

### (E)-3-(5-Brom-furan-2-yl)-allyliden]-malononitril (B2)

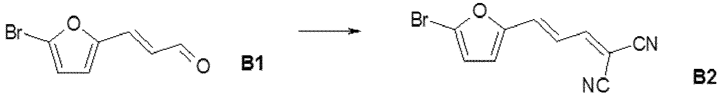

36,7 mmol (E)-3-(5-Brom-furan-2-yl)-propenal und 44,0 mmol Malonitril wurden in 50 ml Ethanol gelöst. 3,7 mmol ß-Alanin wurde dazu gegeben und das Reaktionsgemisch wurde 24 h bei Raumtemperatur gerührt. Der ausgefallene Niederschlag wurde kurz zum Sieden erhitzt und anschließend im Eisbad abgekühlt. Der auskristallisierte Feststoff wurde abfiltriert und mit wenig Ethanol nachgewaschen. Nach dem Trocknen im Exsikkator wurden 3,49 g [(E)-3-(5-Brom-furan-2-yl)-allyliden]-malononitril **B2** (38% Ausbeute) isoliert. El m/z: 250[M], 169, 141, 114.

### (E)-3-(5-Trimethylstannyl-furan-2-yl)-propenal (B3)

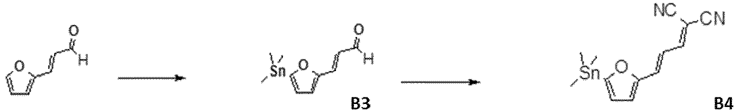

Zu einer Lösung aus 3.06 g (29.9mmol) 1-Methylpiperazin in 82ml wasserfreiem THF wurden unter Argonatmosphäre bei -78°C 12ml (30mmol) n-Butyllithiumlösung (2.5M in Hexan) zugetropft. Nach 15 min Rühren wurden 3.15g (25.0mmol) trans-3-(2-Furyl)acrolein zugetropft. Nach weiteren 15 min Rühren wurden 3.95g (33.7mmol) N,N,N',N'-Tetramethylethylendiamin zugetropft. Nach 15 min Rühren wurden 13.4ml (33.5mmol) n-Butyllithiumlösung (2.5M in Hexan) zugetropft. Die Reaktionsmischung wurde 3h bei -20°C gerührt und anschließend erneut auf -78°C abgekühlt. Bei dieser Temperatur wurden 29.9ml (29.9mmol) einer 1M Lösung von Trimethylzinnchlorid in THF zugegeben und die Mischung anschließend 16h bei R.T. gerührt. Anschließend wurden 100ml Wasser zugegeben, die organische Phase abgetrennt, die wässrige Phase dreimal mit MTBE extrahiert und die vereinigten organischen Phasen mit je 80ml einer 1M Salzsäure, gesättigter Ammoniumchloridlösung und Brine gewaschen. Nach dem Trocknen über Natriumsulfat wurden die Lösungsmittel abdestilliert und der Rückstand chromatographisch gereinigt (SiO2, Petrolether/MTBE 5/1). Ausbeute 5.52g (76%). ¹H-NMR (400 MHz) in Aceton-d6: 0.38 (s, 9H), 6.48 (dd, 1H), 6.84 (d, 1H), 6.97(d, 1H), 7.51(d, 1H), 9.63(d, 1H).

### 2-[(E)-3-(5-Trimethylstannanyl-furan-2-yl)-allyliden]-malononitril (B4)

Unter Argonatmosphäre wurden 9.52g (33.4mmol) **B3** und 2.23g (33.4mmol) Malodinitril in 19ml Ethanol gelöst. 152mg (1.67mmol) Beta-Alanin wurden hinzugegeben und die Mischung 4h bei R.T. gerührt. Anschließend wurde auf Rückflusstemperatur erhitzt und langsam unter Rühren auf 0°C abgekühlt. Der Niederschlag wurde abfiltriert, mit 2ml Ethanol gewaschen und im Vakuum getrocknet: 9.10g (82%) oranger kristalliner Feststoff. ¹H-NMR (400 MHz) in Aceton-d6: 0.41 (s, 9H), 6.90 (d, 1H), 7.07 (m, 2H), 7.46 (d, 1H), 8.01(d, 1H)

### 3-(5-Brom-furan-2-yl)-cyclohex-2-enon (B5)

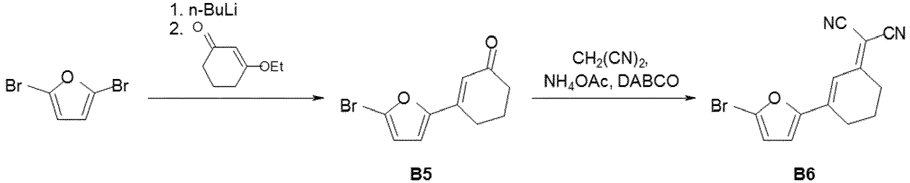

Unter Argonatmosphäre wurde zu einer Lösung aus 2.00g 2,5-Dibromfuran (8.85mmol) in 25mL Diethylether bei -65°C unter Rühren 5.53mL n-Butyllithium (1.6M in Hexan) innerhalb von 15min zugetropft. Nach weiteren 15min wurden 1.86g 3-Ethoxy-2-cyclohexen-1-on (13.3 mmol) zugegeben und die Mischung über Nacht auf R.T. erwärmt. Die Mischung wurde auf 150ml Brine gegeben und mit 3 x 100ml Dichlormethan extrahiert. Die vereinigten organischen Extrakte wurden mit 2M Salzsäure gewaschen, über Natriumsulfat getrocknet und die Lösungsmittel im Vakuum entfernt. Nach säulenchromathographischer Reinigung (SiO₂, Dichlormethan) wurde **B5** als gelber, kristalliner Feststoff erhalten (1.08g, 4.48mmol, 51%). ¹H-NMR (CDCl₃): 6.68 ppm (d, 1H), 6.44-6.43 (m, 2H), 2.60 (td, 2H), 2.46 (t, 2H), 2.14-2.07 (m, 2H).

### 2-[3-(5-Brom-furan-2-yl)-cyclohex-2-enyliden]-malononitril (B6)

Unter Argonatmosphäre wurden 1.68g Ammoniumacetat (21.8mmol) zu einer Lösung aus 1.74 g **B5** (7.14mmol) und 1.42g Malononitril (21.5mmol) in Dichlorethan gegeben. Die Mischung wurde 2h refluxiert, dann 20mg 1,4-diazabicyclo[2.2.2]octane (0.178mmol) zugegeben und anschließend weitere 16h refluxiert. Die Reaktionsmischung wurde auf 100ml Wasser gegeben und mit 3 x 50ml Dichlormethan extrahiert. Die vereinigten organischen Extrakte wurden mit 100ml Wasser gewaschen, über Natriumsulfat getrocknet und die Lösungsmittel im Vakuum entfernt. Nach säulenchromathographischer Reinigung (SiO₂, Hexan) wurde **B6** als oranger, kristalliner Feststoff erhalten (1.15 g, 3.98 mmol, 91%). ¹H-NMR (CDCl₃): 7.19 ppm (s, 1H), 6.79 (d, 1H), 6.49 (d, 1H), 2.80 (t, 2H), 2.64-2.61 (m, 2H), 2.00-1.94 (m, 2H).

### 3-(5-Brom-furan-2-yl)-2-methyl-cyclopent-2-enon (B7)

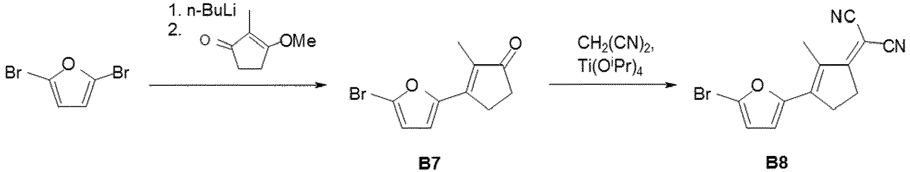

Unter Argonatmosphäre wurden zu einer Lösung aus 3.46g 2,5-Dibromfuran (15mmol) in 45mL Diethylether bei -65°C unter Rühren 6.00mL n-Butyllithium (2.5M in Hexan, 15mmol) innerhalb von 30min zugetropft. Nach weiteren 15min wurden 2.94g 3-Ethoxy-2-methyl-2-cyclopenten-1-on (21.0mmol) gelöst in 15ml Diethylether zugegeben und die Mischung für 1.5h bei -65°C gerührt und anschließend über Nacht auf R.T. erwärmt. Nach der Zugabe von 150ml Dichlormethan wurde die Mischung auf 300ml 1M Salzsäure gegeben. Die organische Phase wurde abgetrennt und die wässrige Phase einmal mit 100ml Dichlormethan extrahiert. Die vereinigten organischen Phasen wurden mit 2M Salzsäure (150ml) und Wasser (100ml) gewaschen, über Natriumsulfat getrocknet und die Lösungsmittel im Vakuum entfernt. Nach säulenchromathographischer Reinigung (SiO₂, Dichlormethan/Hexan) wurde **B7** als gelber, kristalliner Feststoff erhalten (2.10g, 8.71mmol, 58%). ¹H-NMR (CDCl₃): 6.75 ppm (d, 1H), 6.50 (d, 1H), 2.86-2.82 (m, 2H), 2.52-2.49 (m, 2H), 2.02 (t, 3H).

### 2-[3-(5-Brom-furan-2-yl)-2-methyl-cyclopent-2-enyliden]-malononitril (B8)

Eine Lösung aus 1.30g 3-(5-Bromfuran-2-yl)-2-methylcyclopent-2-enon (5.39mmol) und 3.60g Malononitril (53.9 mmol) in 1,2-Dichlorethan wurde unter Argonatmosphäre mit 3.09g Tetraisopropylorthotitanat (10.8 mmol) versetzt und 3d unter Rückfluss gerührt. Die Reaktionsmischung wurde auf Salzsäure (1M, 200mL) gegossen, 30 min heftig gerührt und mit Dichlormethan (3 x 100 mL) extrahiert. Die vereinigten organischen Phasen wurden mit Wasser (100mL) gewaschen, über Natriumsulfat getrocknet, filtriert und das Lösungsmittel unter vermindertem Druck entfernt. Säulenchromatographische Reinigung (Kieselgel, Dichlormethan) lieferte **B8** (1.37 mg, 4.75 mmol, 88%) als orangefarbenen kristallinen Feststoff. ¹H-NMR (CDCl₃): 6.82 ppm (d, 1H), 6.55 (d, 1H), 3.09-3.06 (m, 2H), 3.00-2.96 (m, 2H), 2.40 (t, 3H).

### Synthese der Verbindungen C1-C39

### 2-[5-(1-Methyl-1H-pyrrol-2-yl)-furan-2-ylmethylen]-malononitril (C1)

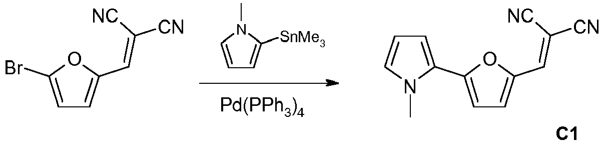

In einem ausgeheizten Schlenkgefäß wurde **C12** (1.01 g, 4.52 mmol) und **A4** (849 mg, 3.48 mmol) in trockenem Tetrahydrofuran (5 mL) unter Argon vorlegt und mit Tetrakis-(triphenylphosphin)-palladium(0) (101 mg, 87 *µ*mol) versetzt. Die Reaktionsmischung wurde 16 h bei 80 °C Badtemperatur gerührt, auf Wasser (ca. 150 mL) gegossen und mit Dichlormethan (3 x 100 mL) extrahiert. Die vereinigten organischen Phasen wurden über Natriumsulfat getrocknet, filtriert und das Lösungsmittel unter vermindertem Druck entfernt. Säulenchromatographische Reinigung (Kieselgel, Dichlormethan/Petrolether) lieferte **C1** (420 mg, 1.88 mmol, 54%) als roten kristallinen Feststoff. ¹H-NMR (Aceton-D₆): 7.84 ppm (s, 1H), 7.52 (d, 1H), 7.03-7.02 (m, 1H), 6.97 (d, 1H), 6.90 (dd, 1H), 6.21 (dd, 1H), 4.00 (s, 3H).

### 2-[5-(1-Propyl-1H-pyrrol-2-yl)-furan-2-ylmethylen]-malononitril (C3)

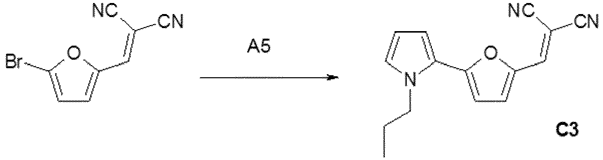

In einem ausgeheizten Schlenkgefäß wurde **C12** (669 mg, 3.00 mmol) und **A5** (1.10 g, 3.00 mmol) in trockenem Tetrahydrofuran (5 mL) unter Argon vorlegt und mit Tetrakis-(triphenylphosphin)-palladium(0) (87 mg, 75 *µ*mol) versetzt. Die Reaktionsmischung wurde 16 h bei 70 °C Badtemperatur gerührt, auf ca. 150 mL Wasser gegossen und mit Dichlormethan (3 x 100 mL) extrahiert. Die vereinigten organischen Phasen wurden über Natriumsulfat getrocknet, filtriert und das Lösungsmittel unter vermindertem Druck entfernt. Säulenchromatographische Reinigung (Kieselgel, Dichlormethan/Petrolether) lieferte **C3** (500 mg, 1.99 mmol, 66%) als orangefarbenes viskoses Öl. ¹H-NMR (Aceton-d₆): 7.85 ppm (s, 1H), 7.53 (d, 1H), 7.10 (dd, 1H), 6.95-6.93 (m, 2H), 6.23 (dd, 1H), 4.36 (t, 2H), 1.75 (sext, 2H), 0.88 (t, 3H).

### 2-[5-(1-Methyl-1H-pyrrol-2-yl)-thiophen-2-ylmethylen]-malononitril (C5)

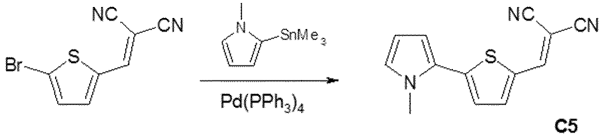

In einem ausgeheizten Schlenkgefäß wurde **C11** (1.09 g, 4.55 mmol) und **A4** in trockenem Tetrahydrofuran (7 mL) unter Argon vorlegt und mit Tetrakis-(triphenylphosphin)-palladium(0) (105 mg, 91 *µ*mol) versetzt. Die Reaktionsmischung wurde 16 h bei 80 °C Badtemperatur gerührt, auf Wasser (ca. 150 mL) gegossen und mit Dichlormethan (3 x 100 mL) extrahiert. Die vereinigten organischen Phasen wurden über Natriumsulfat getrocknet, filtriert und das Lösungsmittel unter vermindertem Druck entfernt. Säulenchromatographische Reinigung (Kieselgel, Dichlormethan/Petrolether) lieferte C5 (670 mg, 2.80 mmol, 80%) als roten kristallinen Feststoff. ¹H-NMR (CDCl₃): 7.74 ppm (s, 1H), 7.66 (d, 1H), 7.19 (d, 1H), 6.84-6.82 (m, 1H), 6.68 (dd, 1H), 6.23 (dd, 1H), 3.87 (s, 3H).

### 2-[5-(1-Ethyl-1H-pyrrol-2-yl)-thiophen-2-ylmethylen]-malononitril (C7)

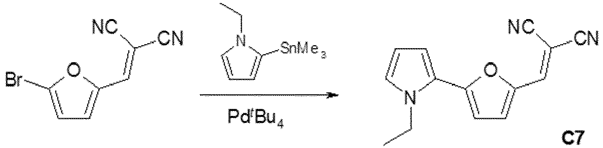

In einem ausgeheizten Schlenkgefäß wurde **C12** (989 mg, 4.43 mmol) und **A6** (1.10 g, 3.41 mmol) in trockenem Dioxan (5 mL) unter Argon vorgelegt, 10 min entgast und mit Tris-(dibenzylidenaceton)-dipalladium(0) (81 mg, 85 *µ*mol) und Tri-*tert*-butylphosphin-tetrafluorborat (100 mg, 341 *µ*mol) versetzt. Die Reaktionsmischung wurde 16 h bei 80 °C Badtemperatur gerührt, auf Wasser (ca. 150 mL) gegossen und mit Dichlormethan (3 x 100 mL) extrahiert. Die vereinigten organischen Phasen wurden über Natriumsulfat getrocknet, filtriert und das Lösungsmittel unter vermindertem Druck entfernt. Säulenchromatographische Reinigung (Kieselgel, Dichlormethan) lieferte C7 (820 mg, 3.46 mmol, 78%) als roten Feststoff. ¹H-NMR (Aceton-D₆): 7.85 ppm (s, 1H), 7.54 (d, 1H), 7.12-7.11 (m, 1H), 6.98 (d, 1H), 6.94-6.93 (m, 1H), 6.25-6.23 (m, 1H), 4.43 (q, 2H), 1.39 (t, 3H).

### 2-[5-(1-Phenyl-1H-pyrrol-2-yl)-furan-2-ylmethylen]-malononitril (C9)

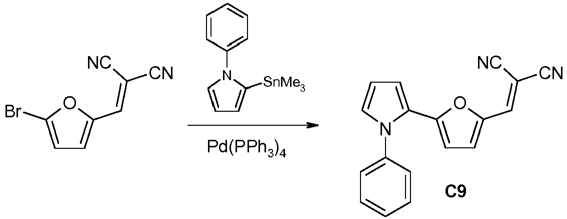

In einem ausgeheizten Schlenkgefäß wurde **C12** (1.45 g, 6.50 mmol) und **A7** (1.91 mg, 5.00 mmol) in trockenem 1,4-Dioxan (7.5 mL) unter Argon vorlegt. Man gibt Tri-tert-butylphosphin-tetrafluorborat (147 mg, 0.50 mmol) und Tris-(dibenzylidenaceton)-dipalladium(0) (118 mg, 125 *µ*mol) hinzu. Die Reaktionsmischung wurde 16 h bei 80 °C Badtemperatur gerührt, auf Wasser (ca. 150 mL) gegossen und mit Dichlormethan (3 x 100 mL) extrahiert. Die vereinigten organischen Phasen wurden über Natriumsulfat getrocknet, filtriert und das Lösungsmittel unter vermindertem Druck entfernt. Säulenchromatographische Reinigung (Kieselgel, Dichlormethan/Petrolether) lieferte 2-[5-(1-Phenyl-1H-pyrrol-2-yl)-furan-2-ylmethylen]malononitril (1.35 g, 4.73 mmol, 94%) als roten kristallinen Feststoff.
1H-NMR (Aceton-D₆): 7.82 ppm (s, 1H), 7.59 (m, 3H), 7.47 (m, 2H), 7.28 (d, 1H), 7.17 (m, 1H), 7.07 (m, 1H), 6.47 (m, 1H), 5.57 (d, 1H).

### 2-(5-Brom-thiophen-2-ylmethylen)-malononitril (C11)

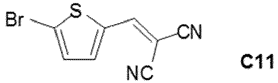

### 2-(5-Brom-furan-2-ylmethylen)-malononitril (C12)

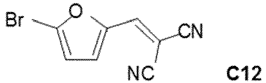

Verbindungen C11 und C12 werden entsprechend der literaturbeschriebenen Synthese (Qi et al., J. Mat. Chem. 2008, 18, 1131) hergestellt

### 2-(5-Furan-2-yl-thiophen-2-ylmethylen)-malononitril (C13)

### 2-[5-(5-Brom-furan-2-yl)-thiophen-2-ylmethylen]-malononitril (C14)

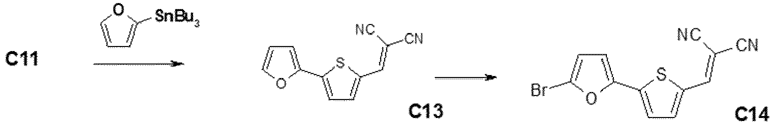

### 2-(5-Furan-2-yl-thiophen-2-ylmethylen)-malononitril (C13)

In einem ausgeheizten Schlenkgefäß wurde **C11** (2.39 g, 10.0 mmol) und 2-Tributylstannylfuran (4.79 g, 13.0 mmol) in trockenem 1,4-Dioxan (14.9 mL) unter Argon vorlegt. Man gibt Tri-tert-butylphosphin-tetrafluorborat (293 mg, 1.00 mmol) und Tris-(dibenzylidenaceton)-dipalladium(O) (236 mg, 250 µmol) hinzu. Die Reaktionsmischung wurde 16 h bei 80 °C Badtemperatur gerührt. Die orangene Suspension wurde filtriert und der Rückstand aus Ethanol umkristallisiert. Man erhielt Produkt **C13** (1.67 g, 4.73 mmol, 74%) als orangen kristallinen Feststoff.
¹H-NMR (CDCl₃): 7.78 ppm (s, 1H), 7.67 (d, 1H), 7.55 (d, 1H), 7.36 (d, 1H), 6.85 (d, 1H), 6.56 (dd, 1H).

### 2-[5-(5-Brom-furan-2-yl)-thiophen-2-ylmethylen]-malononitril (C14)

2-(5-Furan-2-yl-thiophen-2-ylmethylen)-malononitril **(C13)** (1.11 g, 4.86 mmol) wurde unter Argon in trockenem Tetrahydrofuran (44 mL) bei -70 °C vorgelegt und mit N-Bromsuccinimid (874 mg, 4.86 mmol) versetzt. Die Reaktionsmischung wurde unter Lichtausschluss 30 min bei -70°C gerührt und über Nacht im Kältebad allmählich auf Raumtemperatur erwärmt. Nach der Zugabe von 50 ml Wasser wurde das Produkt abfiltriert und getrocknet. Der Rückstand wurde aus Ethanol umkristallisiert und man erhielt das Produkt **C14** (1200 mg, 3.93 mmol, 81%) als orangen kristallinen Feststoff. ¹H-NMR (CDCl₃): 7.78 ppm (s, 1H), 7.66 (d, 1H), 7.35 (d, 1H), 6.79 (d, 1H), 6.49 (d, 1H).

### Allgemeine Vorschrift Bromierung und Stille-Kopplung

1 mmol des entsprechenden Pyrrols wurde in 25 ml trockenem THF gelöst und unter Argon auf-78°C abgekühlt. 0,8 mmol NBS, gelöst in 10 ml trockenem THF, wurde bei -78°C langsam zugetropft und das Reaktionsgemisch 2h bei -78°C gerührt. Anschließend, wurde das Gemisch auf Raumtemperatur gebracht, 35 ml Dioxan, 1,2 mmol 2-(5-Trimethylstannanyl-furan-2-ylmethylene)-malononitril (C18) und 1 mol% Pd[P(t-Bu3)]2 zugegeben. Das Reaktionsgemisch wurde 16 h bei 80°C gerührt. Säulenchromatographische Reinigung (Kieselgel, Dichlormethan) lieferte das entsprechende Produkt

| **Struktur** | **Nummer** | **Edukt** | **Ausbeute** | **Charakterisierung** |
|---|---|---|---|---|
| 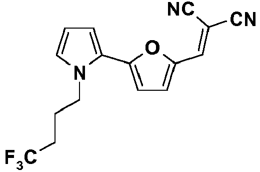 | C26 | A13 | 58% | GC-MS m/z: 319 [M], 222, 180, 104, 77 |
| 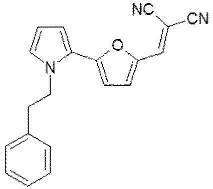 | C24 | A10 | 55% | ¹H-NMR (CDCl3) ppm: 7.28 (s, 1H), 7.20 (m, 5H), 6.96 (d, 2H), 6.82 (d, 1H), 6.62 (d, 2H), 6.18 (d, 1H), 4.60 (t, 2H), 3.03 (t, 2H). |
| 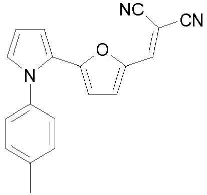 | C22 | A16 | 32% | 1H-NMR (Aceton-d6): 7.82 ppm (s, 1H), 7.41 (m, 2H), 7.34 (m, 2H), 7.25 (d, 1H), 7.13 (m, 1H), 7.05 (m, 1H), 6.45(m, 1H), 5.57 (d, 1H), 2.46 (s, 3H). |
| 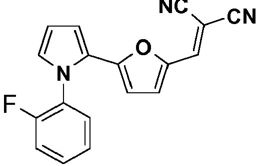 | C36 | A17 | 40% | ¹H-NMR (DMSO-d6): 8.08 ppm (s, 1H), 7.59-7.66 (m, 2H), 7.52 (t, 1H), 7.42 (t, 1H), 7.31 (m, 2H), 6.96 (m, 1H), 6.52 (m, 1H), 5.58 (d, 1H). |
| 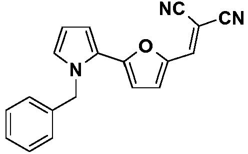 | C38 | A19 | 25% | 1H-NMR (Aceton-d6): 7.82 ppm (s, 1H), 7.41 (m, 1H), 7.30 (m, 2H), 7.23 (m, 2H), 7.06 (m, 2H), 6.99 (d, 1H), 6.76 (d, 1H), 6.34 (d, 1H), 5.65 (s, 2H). |

### Allgemeine Vorschrift für Bromierung

1 mmol Edukt1 wurde unter Argon in trockenem Tetrahydrofuran (10 mL) bei -70 °C vorgelegt und mit N-Bromsuccinimid (178 mg, 1 mmol) versetzt. Die Reaktionsmischung wurde unter Lichtausschluss gerührt und über Nacht im Kältebad allmählich auf Raumtemperatur erwärmt. Nach der Zugabe von Triethylamin (1 mL) wurde das Lösungsmittel unter vermindertem Druck entfernt. Säulenchromatographische Reinigung (Kieselgel, Dichlormethan) lieferte das entsprechende bromierte Produkt.

| **Produkt** | **Edukt1** | | **Ausbeute / %** | **Analytik** |
|---|---|---|---|---|
| **C4** | 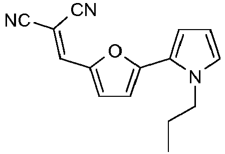 | **C3** | 82 | ¹H-NMR (CDCl₃): 7.31-7.28 ppm (m, 2H), 6.83 (d, 1H), 6.64 (d, 1H), 6.31 (d, 1H), 4.34 (t, 2H), 1.73 (sext, 2H), 0.92 (t, 3H). |
| **C6** | 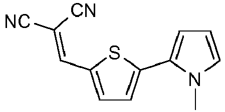 | **C5** | 78 | ¹H-NMR (CDCl₃): 7.75 ppm (s, 1H), 7.68 (d, 1H), 7.16 (d, 1H), 6.61 (d, 1H), 6.31 (d, 1H), 3.81 (s, 3H). |
| **C10** | 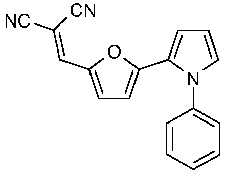 | **C9** | 37 | ¹H-NMR (Aceton-D₆): 7.84 ppm (s, 1H), 7.68-7.70 (m, 3H), 6.46 (m, 2H), 7.23 (d, 1H), 7.04 (d, 1H), 6.60 (d, 1H), 5.27 (d, 1H). |
| **C2** | 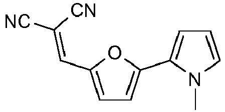 | **C1** | 93 | ¹H-NMR (CDCl₃): 7.30 ppm (s, 1H), 7.26-7.25 (m, 1H), 6.79 (d, 1H), 6.68 (d, 1H), 6.32 (d, 1H), 3.94 (s, 3H). |
| **C8** | 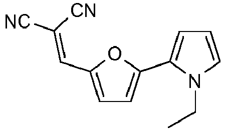 | **C7** | 50 | ¹H-NMR (Aceton-D₆): 7.91 ppm (s, 1H), 7.56 (d, 1H), 7.05 (d, 1H), 6.98 (d, 1H), 6.41 (d, 1H), 4.49 (q, 2H), 1.33 (t, 3H). |
| **C25** | 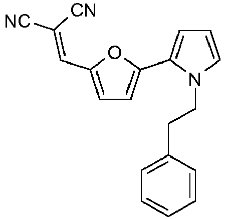 | **C24** | 94 | ¹H-NMR (CDCl3) ppm: 7.93 (s, 1H), 7.57 (d, 1H), 7.08 (d, 1H), 6.99 (d, 1H), 6.44 (d, 1H), 4.59 (t, 2H), 2.35 (m, 2H), 1.99 (m, 2H). |
| **C27** | 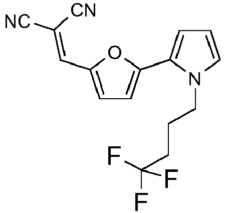 | **C26** | 95 | ¹H-NMR (Aceton-D₆) ppm: 7.28 (s, 1H), 7.17 (m, 4H), 6.96 (d, 2H), 6.74 (d, 1H), 6.49 (d, 1H), 6.32 (d, 1H), 4.64 (t, 2H), 2.97 (m, 2H). |
| **C37** | 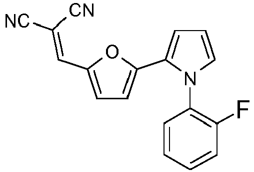 | **C36** | 80 | 1H-NMR (Aceton-D6): 7.86 ppm (s, 1H), 7.75-7.81 (m, 1H), 7.63 (m, 1H), 7.53 (m, 2H), 7.28 (d, 1H), 7.08 (d, 1H), 6.67 (d, 1H), 5.47 (d, 1H). |
| **C23** | 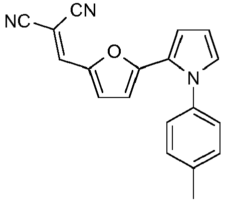 | **C22** | 79 | 1H-NMR (DMSO-D6): 8.09 ppm (s, 1H), 7.45 (d, 2H), 7.34 (d, 2H), 7.25 (d, 1H), 6.89 (d, 1H), 6.66 (d, 1H), 5.20 (d, 1H), 2.45 (s, 3H). |
| **C39** | 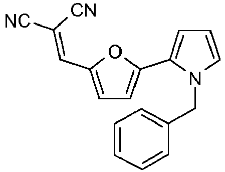 | **C38** | 74 | 1H-NMR (DMSO-D6): 7.99 ppm (s, 1H), 7.44 (d, 1H), 7.31 (m, 2H), 7.24 (m, 1H), 6.98 (m, 3H), 6.84 (d, 1H), 6.55 (d, 1H), 5.62 (s, 2H). |

Die Erfindung ist nicht durch die Beschreibung anhand der Ausführungsbeispiele beschränkt.

## Patentansprüche

1. Verbindungen der allgemeinen Formel I:
EWG1-(T1)ₐ-(T2)_{b},-(Z)_{c}-(T3)_{d}-(T4)ₑ-EWG2
- mit den Parametern a, b, d, e jeweils unabhängig voneinander 0 oder 1,
- mit dem Parameter c= 1, 2, 3, 4, oder 5,
- wobei die allgemeine Gruppe Z ein Block aus zwei Gruppen M und N ist, verknüpft als *-M-N-* oder *-N-M-*, wobei * die Anknüpfung an die Gruppen T1 bis T4 oder EWG1 und EWG2 bezeichnet,
- wobei die Gruppen M jeweils unabhängig voneinander ausgewählt sind aus: 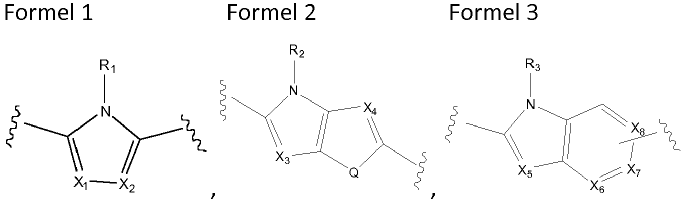
- wobei die Gruppen N jeweils unabhängig voneinander ausgewählt sind aus: 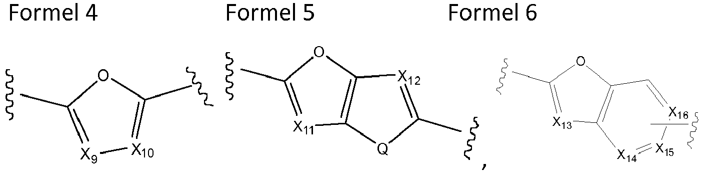
- wobei M und N jeweils derart verknüpft sind, dass mindestens ein N-Atom der Gruppe M und ein O-Atom der Gruppe N jeweils über 2 C-Atome miteinander verbunden sind, und 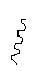 die Anknüpfung an die anderen Gruppen in der Verbindung der allgemeinen Formel I bezeichnet,
- mit X₁-X₁₆ unabhängig voneinander ausgesucht aus N oder C-R, mit der Maßgabe, dass in den Gruppen der Formeln 3 und 6 jeweils eine Gruppe aus den Gruppen X₈/X₇ und X₁₆/X₁₅ die Anknüpfung 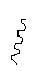 an die anderen Gruppen in der Verbindung der allgemeinen Formel I bezeichnet,
- mit R jeweils unabhängig voneinander ausgesucht aus einer Gruppe aus H, Halogen, verzweigtes oder lineares, zyklisches oder offenkettiges C1-C20 Alkyl, wobei Wasserstoffatome des C₁-C₂₀ Alkyls substituiert sein können und C-Atome des C₁-C₂₀ Alkyls durch Heteroatome ersetzt sein können, C2-C20 Alkenyl, O-Alkyl, S-Alkyl, O-Alkenyl, S-Alkenyl, Alkinyl, Aryl, Heteroaryl, wobei bei allen diesen Gruppen Wasserstoffatome substituiert sein können, CN, NR'R", mit R' und R" jeweils unabhängig voneinander ausgewählt aus: H, verzweigtes oder lineares, zyklisches oder offenkettiges C1-C20 Alkyl, wobei Wasserstoffatome des C₁-C₂₀ Alkyls substituiert sein können und C-Atome des C₁-C₂₀ Alkyls durch Heteroatome ersetzt sein können,
- mit R1-R3 jeweils unabhängig voneinander ausgewählt aus einer Gruppe aus H, verzweigtes oder lineares, zyklisches oder offenkettiges C1-C20 Alkyl, wobei Wasserstoffatome des C₁-C₂₀ Alkyls substituiert sein können, und C-Atome des C₁-C₂₀ Alkyls durch Heteroatome ersetzt sein können, substituiertes oder unsubstituiertes C2-C20 Alkenyl, substituiertes oder unsubstituiertes Aryl, substituiertes oder unsubstituiertes Heteroaryl, CN,
- mit Q jeweils unabhängig voneinander ausgesucht aus S, O, Se, NR'", wobei R'" definiert ist wie R1 bis R3,
- wobei die elektronenziehenden Gruppen EWG1 und EWG2 unabhängig voneinander elektronenziehende Gruppen mit zumindest einer C=C-Doppelbindung sind,
- wobei die Gruppen T1, T2, T3 und T4 jeweils unabhängig voneinander ausgewählt sind aus: 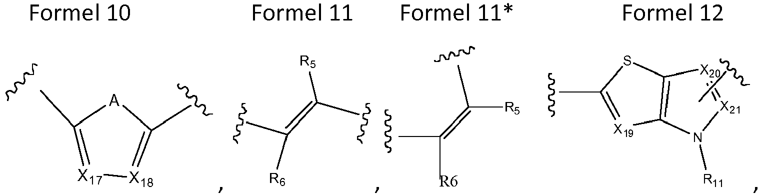 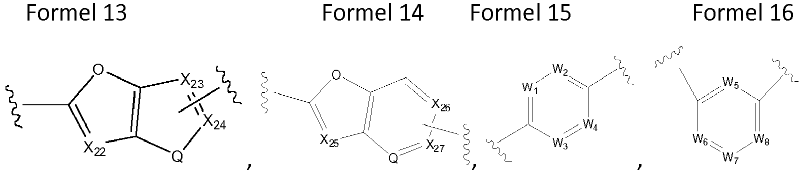
- und 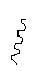 die Anknüpfung an die anderen Gruppen in der Verbindung der allgemeinen Formel I bezeichnet,
- mit R5 und R6 jeweils unabhängig voneinander ausgewählt aus einer Gruppe: H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, wobei, falls der Substituent R13 in der Verbindung der Formel I vorhanden ist, ein Ringschluss zwischen R5 mit R13 oder R6 mit R13 möglich ist, mit der Maßgabe, dass sich zwischen R5 und R13 oder zwischen R6 und R13 jeweils die Doppelbindung aus Formel 11 befindet, möglich ist
- mit W1 bis W8 jeweils unabhängig voneinander ausgewählt aus N, CR, wobei R wie oben beschrieben definiert ist,
- mit X17-X27 unabhängig voneinander ausgesucht aus C-R, wobei R wie oben beschrieben definiert ist und mit der Maßgabe, dass in den Gruppen der Formeln 12, 13 und 14 jeweils eine Gruppe aus den Gruppen X₂₀/X₂₁, X₂₃/X₂₄ und X₂₆/X₂₇ die Anknüpfung 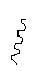 an die anderen Gruppen in der Verbindung der allgemeinen Formel I bezeichnet,
- mit A = S, O, NR"", Se
- mit Q = S, O, NR"", Se
- wobei für die Gruppen A und Q der Substituent R"" jeweils unabhängig voneinander ausgesucht aus H, CN, verzweigtes oder lineares, -zyklisches oder offenkettiges C1-C20 Alkyl, wobei die H-Atome des C1-C20 Alkyl substituiert sein können, C2-C20 Alkenyl, O-Alkyl, S-Alkyl, O-Alkenyl, S-Alkenyl, Alkinyl, substituiertes oder unsubstituiertes Aryl, substituiertes oder unsubstituiertes Heteroaryl.

2. Verbindungen der Formel I nach Anspruch 1, wobei die elektronenziehenden Gruppen EWG1 und EWG2 unabhängig voneinander ausgewählt sind aus: 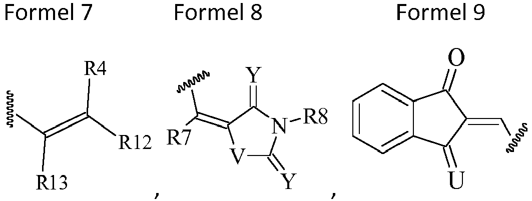
und 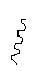 die Anknüpfung an die Gruppen T1 bis T4 oder Z in der Verbindung der allgemeinen Formel I bezeichnet,
mit R4 und R12 jeweils unabhängig voneinander ausgewählt aus H, CN, COOR, mit der Maßgabe, dass R4 und R12 nicht beide H sein können,
wobei R ausgewählt ist aus der gleichen Gruppe von Verbindungen wie bei R1 bis R3 definiert,
mit R13 jeweils unabhängig voneinander ausgewählt aus einer Gruppe: H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, wobei, falls der Substituent R5 oder R6 in der Verbindung der Formel I vorhanden ist, ein Ringschluss zwischen R5 mit R13 oder R6 mit R13 möglich ist, mit der Maßgabe, dass sich zwischen R5 und R13 oder zwischen R6 und R13 jeweils die Doppelbindung aus Formel 11 befindet
mit V= O, S
mit Y= O, S, C(CN)₂
mit U = O, S, C(CN)₂
mit R7 und R8 jeweils unabhängig voneinander ausgewählt aus einer Gruppe H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, wobei für jede der Gruppen EWG1 und EWG2 jeweils für jede C=C-Doppelbindung jeweils unabhängig voneinander sowohl das E-Isomer wie auch das Z-Isomer vorliegen kann.

3. Verbindungen der Formel I nach Anspruch 1 oder 2 mit c = 1 mit der allgemeinen Formel
EWG1 -(T1)ₐ-(T2)_{b}-Z -(T3)_{d}-(T4)ₑ-EWG2

4. Verbindungen der Formel I nach einem der vorhergehenden Ansprüche wobei die elektronenziehenden Gruppen EWG1 oder EWG2 die folgenden Gruppe sind:

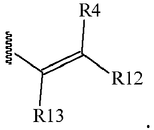

5. Verbindungen nach dem vorhergehenden Patentanspruch, wobei R4 und R12 CN sind.

6. Verbindungen nach einem der vorhergehenden Ansprüche mit der allgemeinen Formel
EWG1-(T1)ₐ-(T2)_{b}-(Z)_{c}-(T3)_{d}-(T4)ₑ-EWG2
mit einem der Indizes a, b, d, e =0.

7. Verbindungen der Formel I nach einem der vorhergehenden Ansprüche mit c=1 der allgemeinen Formel
EWG1 -(T1)ₐ-(T2)_{b}-Z-(T3)_{d}-(T4)ₑ-EWG2
mit Z = *-M-N-* oder *-N-M-* und M eine Gruppe der Formel 1:

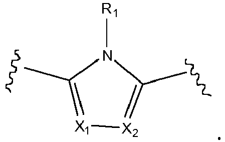

8. Verbindungen der Formel I nach dem vorhergehenden Patentanspruch, wobei X₁ und X₂ unabhängig voneinander ausgewählt aus C-R mit R jeweils unabhängig voneinander ausgesucht aus einer Gruppe aus H, Halogen, verzweigtes oder lineares, zyklisches oder offenkettiges C1-C20 Alkyl.

9. Verbindungen der Formel I nach einem der vorhergehenden Patentansprüche 7 bis 8, wobei die Gruppe N die folgende allgemeine Gruppe der Formel 4 ist:

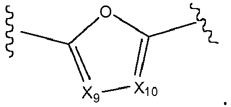

10. Verbindungen nach dem vorhergehenden Patentanspruch, wobei X₉ und X₁₀ unabhängig voneinander ausgewählt aus C-R mit R jeweils unabhängig voneinander ausgesucht aus einer Gruppe aus H, Halogen, verzweigtes oder lineares, zyklisches oder offenkettiges C1-C20 Alkyl.

11. Verbindungen der Formel I nach einem der vorherigen Patentansprüche 6 bis 10, wobei b =1 und T2 die allgemeine Gruppe der Formel 10 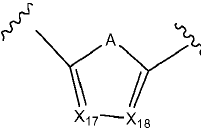 ist.

12. Verbindung der allgemeinen Formel I nach dem vorhergehenden Patentanspruch, wobei in der Formel 10 A = S, O ist.

13. Verbindung nach dem vorhergehenden Patentanspruch mit X₁₇ und X₁₈ = C-R, wobei R unabhängig voneinander ausgewählt aus einer Gruppe aus H, Halogen, verzweigtes oder lineares, zyklisches oder offenkettiges C1-C20 Alkyl.

14. Verbindungen der Formel I nach einem der vorherigen Patentansprüche 6 bis 13, wobei d =1 und die Gruppe T3 ausgewählt ist aus den Gruppen der Formeln 10 oder 11:

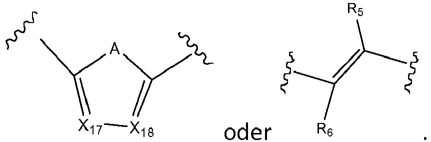

15. Verbindung der allgemeinen Formel I nach dem vorhergehenden Patentanspruch, wobei für die Formel 10 des Patentanspruchs 13 gilt: A = S oder O.

16. Verbindung nach dem vorhergehenden Patentanspruch, wobei X₁₇ und X₁₈ = C-R, wobei R unabhängig voneinander ausgewählt aus einer Gruppe aus H, Halogen, verzweigtes oder lineares, zyklisches oder offenkettiges C1-C20 Alkyl, R5 und R6 jeweils unabhängig voneinander ausgewählt aus H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, wobei falls der Substituent R5 und R6 in der Verbindung vorhanden ist, ein Ringschluss zwischen R5 mit R13 oder R6 mit R13 möglich ist, mit der Maßgabe, dass sich zwischen R5 und R13 oder zwischen R6 und R13 die Doppelbindung aus Formel 11 oder Formel 11* befindet.

17. Verbindung der allgemeinen Formel I nach dem vorhergehenden Patentanspruch, wobei der Ringschluss zwischen den Substituenten R5 mit R13 oder R6 mit R13 in der Form eines gegebenenfalls substituierten Cyclopentenyl-Rings oder eines gegebenenfalls substituierten Cyclohexenyl-Rings vorhanden ist.

18. Verbindungen der Formel I nach einem der vorherigen Patentansprüche 6 bis 17, wobei d =1 und die Gruppe T4 eine Gruppe der Formel 10 oder 11 ist:

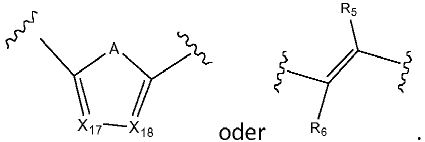

19. Verbindung der allgemeinen Formel I nach dem vorhergehenden Patentanspruch, wobei in der Formel 10 des Anspruchs 15 A = O, S

20. Verbindung nach dem vorhergehenden Patentanspruch, wobei X₁₇ und X₁₈ = C-R, wobei R unabhängig voneinander ausgewählt aus einer Gruppe aus H, Halogen, verzweigtes oder lineares, zyklisches oder offenkettiges C1-C20 Alkyl.

21. Verbindungen nach einem der vorherigen Patentansprüche 6 bis 15, wobei a =1 und die Gruppe T1 ausgewählt ist aus den Gruppen der Formeln 10 und/oder 11:

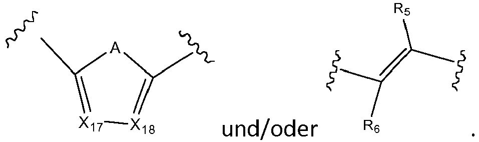

22. Verbindungen nach dem vorhergehenden Patentanspruch, wobei für die Formel 10 des Patentanspruchs 16 gilt: A = S oder O.

23. Verbindungen nach dem vorhergehenden Patentanspruch, wobei X₁₇ und X₁₈ = C-R, wobei R unabhängig voneinander ausgewählt ist aus einer Gruppe aus H, Halogen, verzweigtes oder lineares, zyklisches oder offenkettiges C1-C20 Alkyl, R5 und R6 jeweils unabhängig voneinander ausgewählt aus H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, wobei falls der Substituent R5 und R6 in der Verbindung vorhanden ist, ein Ringschluss zwischen R5 mit R13 als auch zwischen R6 mit R13 möglich ist, mit der Maßgabe, dass sich zwischen R5 und R13 oder zwischen R6 und R13 die Doppelbindung aus Formel 11 oder Formel 11* befindet.

24. Verbindungen nach Anspruch 1 oder 2 mit folgender allgemeiner Strukturformel II:

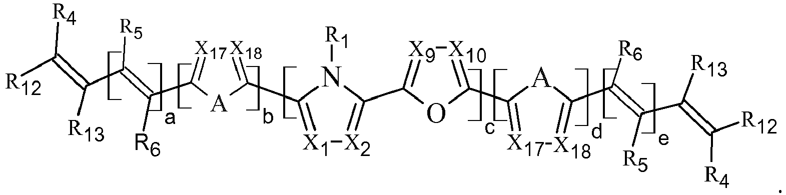

25. Verbindungen nach Anspruch 24, wobei unabhängig voneinander für A gilt A = O, S.

26. Verbindungen nach einem der Ansprüche 24 oder 25, wobei unabhängig voneinander X₁₇, X₁₈, X₁, X₂, X₉ und X₁₀ = C-R, wobei R unabhängig voneinander ausgewählt aus einer Gruppe aus H, Halogen, verzweigtes oder lineares, zyklisches oder offenkettiges C1-C20 Alkyl, R5 und R6 jeweils unabhängig voneinander ausgewählt aus H, CN, F, Aryl, Heteroaryl, C2-C20 Alkenyl, Alkinyl, verzweigtes oder lineares, zyklisches oder offenkettiges C1-C20 Alkyl, wobei Wasserstoffatome des C₁-C₂₀ Alkyls substituiert sein können, und wobei falls die Substituenten R5 und R6 vorhanden sind, einen Ringschluss zwischen R5 mit R13 oder R6 mit R13 bilden können.

27. Verbindungen nach einem der vorherigen Ansprüche 24 bis 26, wobei c = 1.

28. Verbindungen nach einem der vorherigen Ansprüche 24 bis 27, wobei b = 1.

29. Verbindungen nach einem der vorherigen Ansprüche 24 bis 28, wobei e = 1.

30. Verbindungen nach einem der vorherigen Ansprüche 24 bis 29, wobei R4 und R12 jeweils unabhängig voneinander ausgewählt sind aus H, CN, mit der Maßgabe, dass R4 und R12 nicht beide H sein können.

31. Verwendung einer oder mehrerer Verbindungen nach den Ansprüchen 1-30 in einem organischen elektronischen Bauelement.

32. Verwendung nach Anspruch 31 in einem organischen photoaktiven Element, bevorzugt in einer Solarzelle.

33. Organische elektronische Vorrichtung umfassend eine oder mehrere Verbindungen nach einem der Patentansprüche 1 bis 30.

34. Organisches elektronische Vorrichtung nach dem vorhergehenden Patentanspruch umfassend eine Elektrode und eine Gegenelektrode und mindestens eine organische photoaktive Schicht zwischen der Elektrode und der Gegenelektrode, wobei die organische photoaktive Schicht zumindest eine der Verbindungen gemäß einem der Ansprüche 1-30 aufweist.

## Claims

1. Compounds of the general formula I:
EWG1 -(T1)ₐ-(T2)_{b}-(Z)_{c}-(T3)_{d}-(T4),-EWG2
- with the parameters a, b, d and e being each independently of one another 0 or 1,
- with the parameter c being 1, 2, 3, 4 or 5,
- where the general group Z is a block of two groups M and N, linked as *-M-N-* or *-N-M-*, where * designates the attachment to the groups T1 to T4 or EWG1 and EWG2,
- where the groups M each independently of one another are selected from: 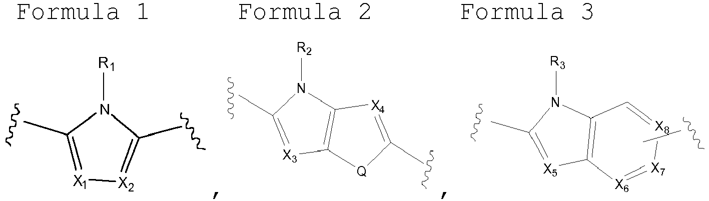
- where the groups N each independently of one another are selected from: 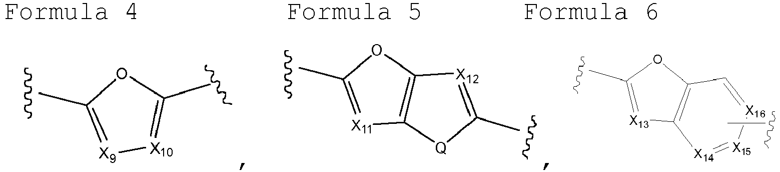
- where M and N are each linked such that at least one N atom of the group M and one O atom of the group N are each joined to one another via 2 C atoms, and 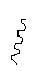 designates the attachment to the other groups in the compound of the general formula I,
- with X₁-X₁₆ independently of one another being selected from N or C-R, with the proviso that in the groups of the formulae 3 and 6, in each case one group from the groups X₈/X₇ and X_{16/}X₁₅ designates the attachment 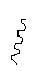 to the other groups in the compound of the general formula I,
- with each R independently of any other being selected from a group composed of H, halogen, branched or linear, cyclic or open-chain C₁-C₂₀ alkyl, where hydrogen atoms of the C₁-C₂₀ alkyl may be substituted and C atoms of the C₁-C₂₀ alkyl may be replaced by heteroatoms; C₂-C₂₀ alkenyl, O-alkyl, S-alkyl, O-alkenyl, S-alkenyl, alkynyl, aryl, heteroaryl, it being possible for hydrogen atoms to be substituted in all of these groups; CN, NR'R", with R' and R" each independently of one another being selected from: H, branched or linear, cyclic or open-chain C₁-C₂₀ alkyl, where hydrogen atoms of the C₁-C₂₀ alkyl may be substituted and C atoms of the C₁-C₂₀ alkyl may be replaced by heteroatoms,
- where R₁ to R₃ each independently of one another are selected from a group composed of H, branched or linear, cyclic or open-chain C₁-C₂₀ alkyl, where hydrogen atoms of the C₁-C₂₀ alkyl may be substituted and C atoms of the C₁-C₂₀ alkyl may be replaced by heteroatoms; substituted or unsubstituted C₂-C₂₀ alkenyl, substituted or unsubstituted aryl, substituted or unsubstituted heteroaryl, CN,
- with each Q independently of any other being selected from S, O, Se, NR"', where R"' is defined as for R₁ to R₃,
- where the electron-withdrawing groups EWG1 and EWG2 independently of one another are electron-withdrawing groups having at least one C=C double bond,
- where the groups T1, T2, T3 and T4 each independently of one another are selected from: 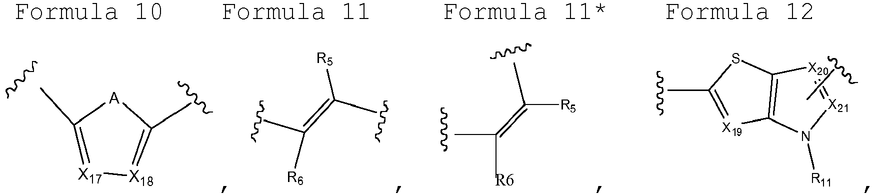 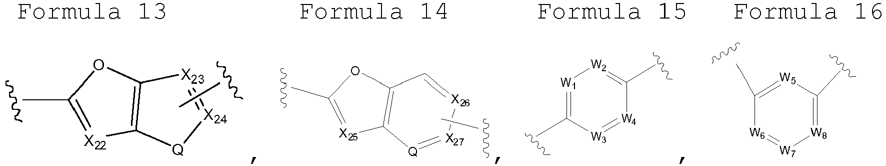
- and 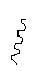 designates the attachment to the other groups in the compound of the general formula I,
- with R5 and R6 each independently of one another being selected from a group: H, CN, F, aryl, heteroaryl, C₂-C₂₀ alkenyl, alkynyl, branched or linear, cyclic or open-chain C₁-C₂₀ alkyl, where hydrogen atoms of the C₁-C₂₀ alkyl may be substituted, where, if the substituent R₁₃ is present in the compound of the formula I, a ring closure between R₅ with R₁₃ or R₆ with R₁₃ is possible, with the proviso that between R₅ and R₁₃ or between R₆ and R₁₃ in each case the double bond from formula 11 is located, is possible,
- with W₁ to W₈ each independently of one another being selected from N, CR, where R is defined as described above,
- with X₁₇ to X₂₇ independently of one another being selected from C-R, where R is defined as described above, and with the proviso that in the groups of the formulae 12, 13 and 14, in each case one group from the groups X₂₀/X₂₁, X₂₃/X₂₄ and X₂₆/X₂₇ designates the attachment 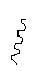 to the other groups in the compound of the general formula I,
- with A being S, O, NR"", Se
- with Q being S, O, NR"", Se
- where for the groups A and Q, the substituent R"" in each case independently of any other is selected from H, CN, branched or linear, cyclic or open-chain C₁-C₂₀ alkyl, where the H atoms of the C₁-C₂₀ alkyl may be substituted; C₂-C₂₀ alkenyl, O-alkyl, S-alkyl, O-alkenyl, S-alkenyl, alkynyl, substituted or unsubstituted aryl, substituted or unsubstituted heteroaryl.

2. The compounds of the formula I as claimed in claim 1, where the electron-withdrawing groups EWG1 and EWG2 independently of one another are selected from: 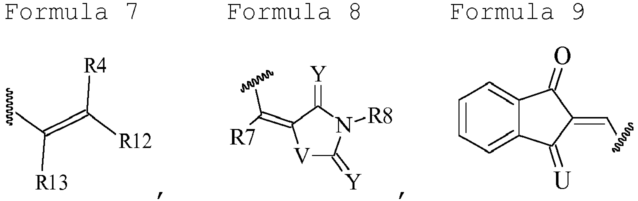
and 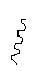 designates the attachment to the groups T1 to T4 or Z in the compound of the general formula I,
with R₄ and R₁₂ each independently of one another being selected from H, CN, COOR, with the proviso that R₄ and R₁₂ cannot both be H,
where R is selected from the same group of compounds as defined in the case of R₁ to R₃,
with each R₁₃ independently of any other being selected from a group: H, CN, F, aryl, heteroaryl, C₂-C₂₀ alkenyl, alkynyl, branched or linear, cyclic or open-chain C₁-C₂₀ alkyl, where hydrogen atoms of the C₁-C₂₀ alkyl may be substituted, where, if the substituent R₅ or R₆ is present in the compound of the formula I, a ring closure between R₅ with R₁₃ or R₆ with R₁₃ is possible, with the proviso that the double bond from formula 11 is located in each case between R₅ and R₁₃ or between R₆ and R₁₃,
with V being O, S; with Y being O, S, C(CN)₂; with U being O, S, C(CN)₂,
with R₇ and R₈ each independently of one another being selected from a group H, CN, F, aryl, heteroaryl, C₂-C₂₀ alkenyl, alkynyl, branched or linear, cyclic or open-chain C₁-C₂₀ alkyl, where hydrogen atoms of the C₁-C₂₀ alkyl may be substituted, where for each of the groups EWG1 and EWG2, each independently of one another in each case for each C=C double bond, both the E-isomer and the Z-isomer may be present.

3. The compounds of the formula I as claimed in claim 1 or 2 with c being 1, having the general formula
EWG1 -(T1)ₐ-(T2)_{b}-Z-(T3)_{d}-(T4)ₑ-EWG2

4. The compounds of the formula I as claimed in any of the preceding claims, wherein the electron-withdrawing groups EWG1 or EWG2 are the following group:

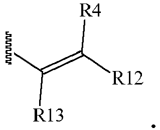

5. The compounds as claimed in the preceding claim, where R₄ and R₁₂ are CN.

6. The compounds as claimed in any of the preceding claims, having the general formula
EWG1 -(T1)ₐ-(T2)_{b}-(Z)_{c}-(T3)_{d}-(T4),-EWG2
with one of the indices a, b, d and e being 0.

7. The compounds of the formula I as claimed in any of the preceding claims, with c = 1, of the general formula
EWG1 -(T1),-(T2)_{b}-Z-(T3)_{d}-(T4),-EWG2
with Z = *-M-N-* or *-N-M-* and M being a group of the formula 1:

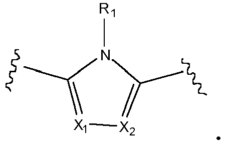

8. The compounds of the formula I as claimed in the preceding claim, where X₁ and X₂ independently of one another are selected from C-R with each R independently of any other selected from a group composed of H, halogen, branched or linear, cyclic or open-chain C₁-C₂₀ alkyl.

9. The compounds of the formula I as claimed in either of the preceding claims 7 to 8, where the group N is the following general group of the formula 4:

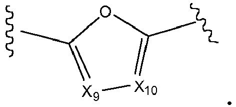

10. The compounds as claimed in the preceding claim, where X₉ and X₁₀ independently of one another are selected from C-R with each R independently of any other being selected from a group composed of H, halogen, branched or linear, cyclic or open-chain C₁-C₂₀ alkyl.

11. The compounds of the formula I as claimed in any of preceding claims 6 to 10, where b is 1 and T2 is the general group of the formula 10

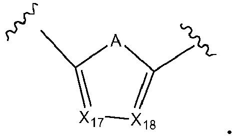

12. The compound of the general formula I as claimed in the preceding claim, wherein A in the formula 10 is S or O.

13. The compound as claimed in the preceding claim, with X₁₇ and X₁₈ being C-R, where R independently at each occurrence is selected from a group composed of H, halogen, branched or linear, cyclic or open-chain C₁-C₂₀ alkyl.

14. The compounds of the formula I as claimed in any of preceding claims 6 to 13, where d = 1 and the group T3 is selected from the groups of the formulae 10 or 11:

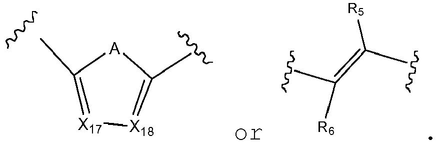

15. The compound of the general formula I as claimed in the preceding claim, where for the formula 10 of claim 13 it is the case that: A = S or O.

16. The compound as claimed in the preceding claim, where X₁₇ and X₁₈ are C-R, where R independently at each occurrence is selected from a group composed of H, halogen, branched or linear, cyclic or open-chain C₁-C₂₀ alkyl, R₅ and R₆ each independently of one another are selected from H, CN, F, aryl, heteroaryl, C₂-C₂₀ alkenyl, alkynyl, branched or linear, cyclic or open-chain C₁-C₂₀ alkyl, where hydrogen atoms of the C₁-C₂₀ alkyl may be substituted, where, if the substituent R₅ and R₆ is present in the compound, a ring closure is possible between R₅ with R₁₃ or R₆ with R₁₃, with the proviso that the double bond from formula 11 or formula 11* is located between R₅ and R₁₃ or between R₆ and R₁₃.

17. The compound of the general formula I as claimed in the preceding claim, where the ring closure between the substituents R₅ with R₁₃ or R₆ with R₁₃ is present in the form of an optionally substituted cyclopentenyl ring or of an optionally substituted cyclohexenyl ring.

18. The compounds of the formula I as claimed in any of preceding claims 6 to 17, where d = 1 and the group T4 is a group of the formula 10 or 11:

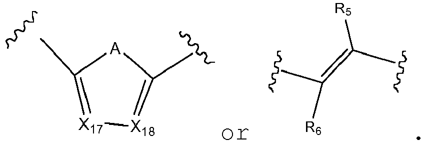

19. The compound of the general formula I as claimed in the preceding claim, where A = O or S in the formula 10 of claim 15.

20. The compound as claimed in the preceding claim, where X₁₇ and X₁₈ are C-R, where R is selected independently of any other from a group composed of H, halogen, branched or linear, cyclic or open-chain C₁-C₂₀ alkyl.

21. The compounds as claimed in any of preceding claims 6 to 15, where a = 1 and the group T1 is selected from the groups of the formulae 10 and/or 11:

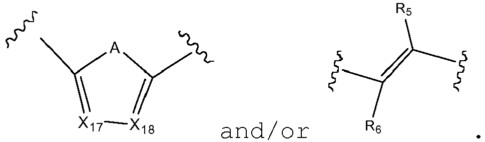

22. The compounds as claimed in the preceding claim, where A = S or O for the formula 10 in claim 16.

23. The compounds as claimed in the preceding claim, where X₁₇ and X₁₈ are C-R, where R independently at each occurrence is selected from a group composed of H, halogen, branched or linear, cyclic or open-chain C₁-C₂₀ alkyl, R5 and R6 are each selected independently of one another from H, CN, F, aryl, heteroaryl, C₂-C₂₀ alkenyl, alkynyl, branched or linear, cyclic or open-chain C₁-C₂₀ alkyl, where hydrogen atoms of the C₁-C₂₀ alkyl may be substituted, where, if the substituent R₅ and R₆ is present in the compound, a ring closure between R₅ with R₁₃ and also between R₆ with R₁₃ is possible, with the proviso that the double bond from formula 11 or formula 11* is located between R₅ and R₁₃ or between R₆ and R₁₃.

24. The compounds as claimed in claim 1 or 2, having the following general structural formula II:

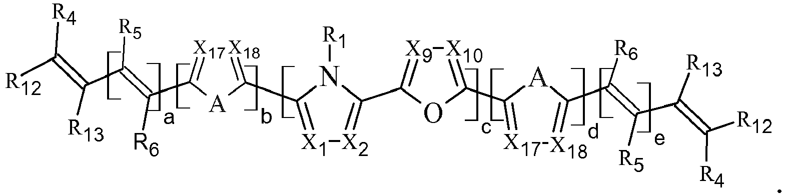

25. The compounds as claimed in claim 24, where A is O or S independently of one another for A.

26. The compounds as claimed in either of claims 24 and 25, where independently of one another X₁₇, X₁₈, X₁, X₂, X₉ and X₁₀ are C-R, where R independently at each occurrence is selected from a group composed of H, halogen, branched or linear, cyclic or open-chain C₁-C₂₀ alkyl, R₅ and R₆ each independently of one another are selected from H, CN, F, aryl, heteroaryl, C₂-C₂₀ alkenyl, alkynyl, branched or linear, cyclic or open-chain C₁-C₂₀ alkyl, where hydrogen atoms of the C₁-C₂₀ alkyl may be substituted, and where, if the substituents R₅ and R₆ are present, a ring closure may be formed between R₅ with R₁₃ or R₆ with R₁₃.

27. The compounds as claimed in any of preceding claims 24 to 26, where c is 1.

28. The compounds as claimed in any of preceding claims 24 to 27, where b is 1.

29. The compounds as claimed in any of preceding claims 24 to 28, where e is 1.

30. The compounds as claimed in any of preceding claims 24 to 29, where R₄ and R₁₂ each independently of one another are selected from H, CN, with the proviso that R4 and R12 cannot both be H.

31. The use of one or more compounds as claimed in claims 1 to 30 in an organic electronic component.

32. The use as claimed in claim 31 in an organic photoactive element, preferably in a solar cell.

33. An organic electronic device comprising one or more compounds as claimed in any of claims 1 to 30.

34. The organic electronic device as claimed in the preceding claim, comprising an electrode and a counterelectrode and at least one organic photoactive layer between the electrode and the counterelectrode, where the organic photoactive layer comprises at least one of the compounds as claimed in any of claims 1 to 30.

## Revendications

1. Composés de formule générale I :
EWG1-(T1)ₐ-(T2)_{b}-(Z)-(T3)_{d}-(T4)-EWG2
- avec les paramètres a, b, d, e chacun indépendamment les uns des autres 0 ou 1,
- avec le paramètre c = 1, 2, 3, 4 ou 5,
- le groupe général Z étant une séquence de deux groupes M et N, reliés selon *-M-N-* ou *-N-M-*, * désignant la liaison aux groupes T1 à T4 ou EWG1 et EWG2,
- les groupes M étant chacun choisis indépendamment les uns des autres parmi : 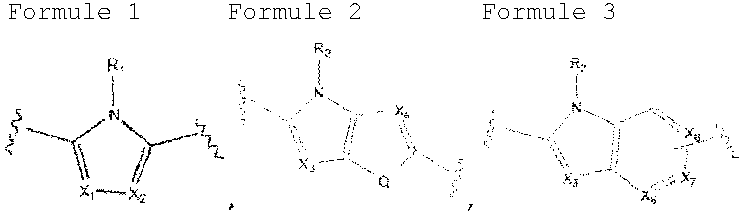
- les groupes N étant chacun choisis indépendamment les uns des autres parmi : 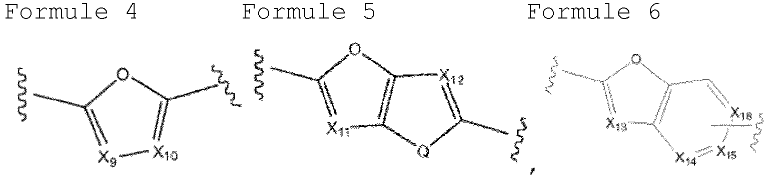
- M et N étant chacun reliés de telle sorte qu'au moins un atome N du groupe M et un atome O du groupe N soient reliés l'un avec l'autre par 2 atomes C, et 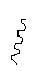 désignant la liaison aux autres groupes dans le composé de formule générale I,
- X₁ à X₁₆ étant choisis indépendamment les uns des autres parmi N ou C-R, à condition que dans les groupes des formules 3 et 6, un groupe parmi les groupes X₈/X₇ et X_{16/}X₁₅ désigne la liaison 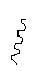 aux autres groupes dans le composé de formule générale I,
- les R étant chacun choisis indépendamment les uns des autres dans le groupe constitué par H, halogène, alkyle en C1-C20 ramifié ou linéaire, cyclique ou à chaîne ouverte, des atomes d'hydrogène de l'alkyle en C₁-C₂₀ pouvant être substitués et des atomes C de l'alkyle en C₁-C₂₀ pouvant être remplacés par des hétéroatomes, alcényle en C2-C20, O-alkyle, S-alkyle, O-alcényle, S-alcényle, alcynyle, aryle, hétéroaryle, des atomes d'hydrogène dans tous ces groupes pouvant être substitués, CN, NR'R", R' et R" étant chacun choisis indépendamment l'un de l'autre parmi : H, alkyle en C1-C20 ramifié ou linéaire, cyclique ou à chaîne ouverte, des atomes d'hydrogène de l'alkyle en C₁-C₂₀ pouvant être substitués et des atomes C de l'alkyle en C₁-C₂₀ pouvant être remplacés par des hétéroatomes,
- R1 à R3 étant chacun choisis indépendamment les uns des autres dans un groupe constitué par H, alkyle en C1-C20 ramifié ou linéaire, cyclique ou à chaîne ouverte, des atomes d'hydrogène de l'alkyle en C₁-C₂₀ pouvant être substitués et des atomes C de l'alkyle en C₁-C₂₀ pouvant être remplacés par des hétéroatomes, alcényle en C2-C20 substitué ou non substitué, aryle substitué ou non substitué, hétéroaryle substitué ou non substitué, CN,
- les Q étant chacun choisis indépendamment les uns des autres parmi S, O, Se, NR"', R"' étant défini tel que R1 à R3,
- les groupes attracteurs d'électrons EWG1 et EWG2 étant indépendamment l'un de l'autre des groupes attracteurs d'électrons contenant au moins une double liaison C=C,
- les groupes T1, T2, T3 et T4 étant chacun choisis indépendamment les uns des autres parmi : 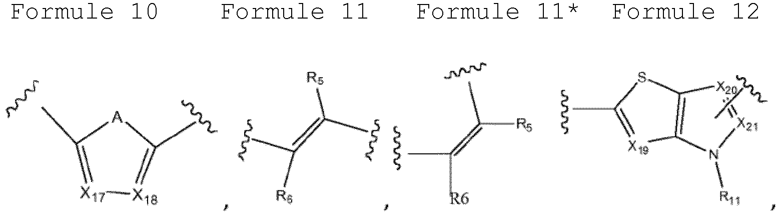 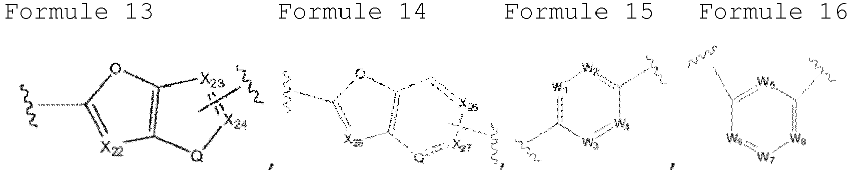
- et 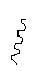 désignant la liaison aux autres groupes dans le composé de formule générale I,
- R5 et R6 étant chacun choisis indépendamment l'un de l'autre dans un groupe constitué par : H, CN, F, aryle, hétéroaryle, alcényle en C2-C20, alcynyle, alkyle en C1-C20 ramifié ou linéaire, cyclique ou à chaîne ouverte, des atomes d'hydrogène de l'alkyle en C₁-C₂₀ pouvant être substitués, si le substituant R13 dans le composé de formule I est présent, une fermeture de cycle entre R5 et R13 ou R6 et R13 étant possible, à condition que la double liaison de la formule 11 se trouve à chaque fois entre R5 et R13 ou entre R6 et R13,
- W1 à W8 étant chacun choisis indépendamment les uns des autres parmi N, CR, R étant tel que défini précédemment,
- X17 à X27 étant choisis indépendamment les uns des autres parmi C-R, R étant tel que défini précédemment, et à condition que, dans les groupes des formules 12, 13 et 14, à chaque fois un groupe parmi les groupes X₂₀/X₂₁, X₂₃/X₂₄ et X₂₆/X₂₇ désigne la liaison 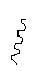 aux autres groupes dans le composé de formule générale I,
- avec A = S, O, NR"", Se,
- avec Q = S, O, NR"", Se,
- pour les groupes A et Q, les substituants R"" étant chacun choisis indépendamment les uns des autres parmi H, CN, alkyle en C1-C20 ramifié ou linéaire, cyclique ou à chaîne ouverte, les atomes H de l'alkyle en C1-C20 pouvant être substitués, alcényle en C2-C20, O-alkyle, S-alkyle, O-alcényle, S-alcényle, alcynyle, aryle substitué ou non substitué, hétéroaryle substitué ou non substitué.

2. Composés de formule I selon la revendication 1, dans lesquels les groupes attracteurs d'électrons EWG1 et EWG2 sont choisis indépendamment les uns des autres parmi : 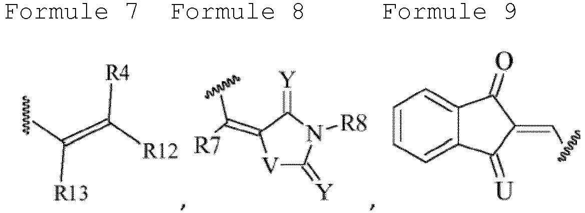
et 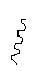 désigne la liaison aux groupes T1 à T4 ou Z dans le composé de formule générale I,
R4 et R12 pouvant chacun indépendamment l'un de l'autre être choisis parmi H, CN, COOR, à condition que R4 et R12 ne puissent pas tous les deux représenter H,
R étant choisi dans le même groupe de composés que R1 à R3,
les R13 étant chacun indépendamment les uns des autres choisis dans un groupe constitué par : H, CN, F, aryle, hétéroaryle, alcényle en C2-C20, alcynyle, alkyle en C1-C20 ramifié ou linéaire, cyclique ou à chaîne ouverte, des atomes d'hydrogène de l'alkyle en C₁-C₂₀ pouvant être substitués, si le substituant R5 ou R6 dans le composé de formule I est présent, une fermeture de cycle étant possible entre R5 et R13 ou entre R6 et R13, à condition que la double liaison de la formule 11 se trouve à chaque fois entre R5 et R13 ou entre R6 et R13,
avec V = O, S,
avec Y = O, S, C(CN)₂,
avec U = O, S, C(CN)₂,
R7 et R8 étant chacun choisis indépendamment l'un de l'autre dans un groupe constitué par H, CN, F, aryle, hétéroaryle, alcényle en C2-C20, alcynyle, alkyle en C1-C20 ramifié ou linéaire, cyclique ou à chaîne ouverte, des atomes d'hydrogène de l'alkyle en C₁-C₂₀ pouvant être substitués, pour chacun des groupes EWG1 et EWG2, pour chaque double liaison C=C, aussi bien l'isomère E que l'isomère Z pouvant être présents indépendamment l'un de l'autre.

3. Composés de formule I selon la revendication 1 ou 2, avec c = 1, de formule générale
EWG1-(T1)a-(T2)b-Z-(T3)d-(T4)e-EWG2

4. Composés de formule I selon l'une quelconque des revendications précédentes, dans lesquels les groupes attracteurs d'électrons EWG1 ou EWG2 sont le groupe suivant :

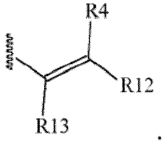

5. Composés selon la revendication précédente, dans lesquels R4 et R12 représentent CN.

6. Composés selon l'une quelconque des revendications précédentes, de formule générale
EWG1-(T1)a-(T2)b-(Z)c-(T3)d-(T4)e-EWG2
avec un des indices a, b, d, e = 0.

7. Composés de formule I selon l'une quelconque des revendications précédentes, avec c = 1, de formule générale
EWG1-(T1)a-(T2)b-Z-(T3)d-(T4)e-EWG2
avec Z = *-M-N-* ou *-N-M-* et M représentant un groupe de formule 1 :

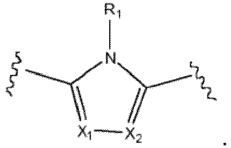

8. Composés de formule I selon la revendication précédente, dans lesquels X₁ et X2 sont choisis indépendamment l'un de l'autre parmi C-R avec les R chacun indépendamment les uns des autres choisis dans un groupe constitué par H, halogène, alkyle en C1-C20 ramifié ou linéaire, cyclique ou à chaîne ouverte.

9. Composés de formule I selon l'une quelconque des revendications 7 à 8 précédentes, dans lesquels le groupe N est le groupe général suivant de formule 4 :

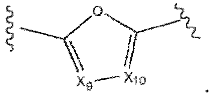

10. Composés selon la revendication précédente, dans lesquels X9 et X10 sont choisis indépendamment l'un de l'autre parmi C-R avec les R chacun indépendamment les uns des autres choisis dans un groupe constitué par H, halogène, alkyle en C1-C20 ramifié ou linéaire, cyclique ou à chaîne ouverte.

11. Composés de formule I selon l'une quelconque des revendications 6 à 10 précédentes, dans lesquels b = 1 et T2 est le groupe général de formule 10

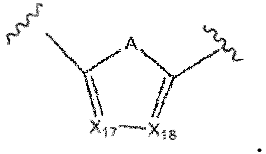

12. Composé de formule générale I selon la revendication précédente, dans lequel, dans la formule 10, A = S, O.

13. Composé selon la revendication précédente, avec X17 et X18 = C-R, les R étant choisis indépendamment les uns des autres dans un groupe constitué par H, halogène, alkyle en C1-C20 ramifié ou linéaire, cyclique ou à chaîne ouverte.

14. Composés de formule I selon l'une quelconque des revendications 6 à 13 précédentes, dans lesquels d = 1 et le groupe T3 est choisi parmi les groupes de formules 10 ou 11 :

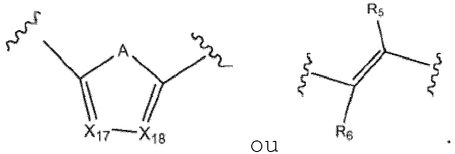

15. Composé de formule générale I selon la revendication précédente, dans lequel, pour la formule 10 de la revendication 13 : A = S ou O.

16. Composé selon la revendication précédente, dans lequel X17 et X18 = C-R, les R étant choisis indépendamment les uns des autres dans un groupe constitué par H, halogène, alkyle en C1-C20 ramifié ou linéaire, cyclique ou à chaîne ouverte, R5 et R6 étant chacun indépendamment l'un de l'autre choisis parmi H, CN, F, aryle, hétéroaryle, alcényle en C2-C20, alcynyle, alkyle en C1-C20 ramifié ou linéaire, cyclique ou à chaîne ouverte, des atomes d'hydrogène de l'alkyle en C1-C20 pouvant être substitués, si les substituants R5 et R6 sont présents dans le composé, une fermeture de cycle entre R5 et R13 ou entre R6 et R13 étant possible, à condition que la double liaison de la formule 11 ou de la formule 11* se trouve entre R5 et R13 ou entre R6 et R13.

17. Composé de formule générale I selon la revendication précédente, dans lequel la fermeture de cycle entre les substituants R5 et R13 ou R6 et R13 est présente sous la forme d'un cycle cyclopentényle éventuellement substitué ou d'un cycle cyclohexényle éventuellement substitué.

18. Composés de formule I selon l'une quelconque des revendications 6 à 17 précédentes, dans lesquels d = 1 et le groupe T4 est un groupe de formule 10 ou 11 :

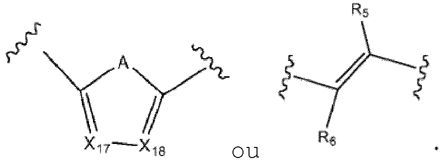

19. Composé de formule générale I selon la revendication précédente, dans lequel, dans la formule 10 de la revendication 15, A = O, S.

20. Composé selon la revendication précédente, dans lequel X17 et X18 = C-R, les R étant choisis indépendamment les uns des autres dans un groupe constitué par H, halogène, alkyle en C1-C20 ramifié ou linéaire, cyclique ou à chaîne ouverte.

21. Composés selon l'une quelconque des revendications 6 à 15 précédentes, dans lesquels a = 1 et le groupe T1 est choisi parmi les groupes de formules 10 et/ou 11 :

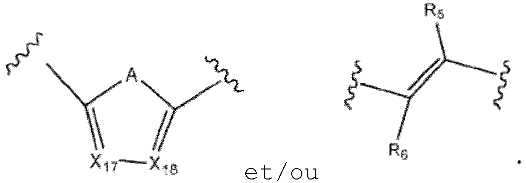

22. Composés selon la revendication précédente, dans lesquels, pour la formule 10 de la revendication 16 : A = S ou O.

23. Composés selon la revendication précédente, dans lesquels X17 et X18 = C-R, les R étant choisis indépendamment les uns des autres dans un groupe constitué par H, halogène, alkyle en C1-C20 ramifié ou linéaire, cyclique ou à chaîne ouverte, R5 et R6 étant chacun indépendamment l'un de l'autre choisis parmi H, CN, F, aryle, hétéroaryle, alcényle en C2-C20, alcynyle, alkyle en C1-C20 ramifié ou linéaire, cyclique ou à chaîne ouverte, des atomes d'hydrogène de l'alkyle en C1-C20 pouvant être substitués, si les substituants R5 et R6 sont présents dans le composé, une fermeture de cycle entre R5 et R13 ou entre R6 et R13 étant possible, à condition que la double liaison de la formule 11 ou de la formule 11* se trouve entre R5 et R13 ou entre R6 et R13.

24. Composés selon la revendication 1 ou 2, ayant la formule structurale générale II suivante :

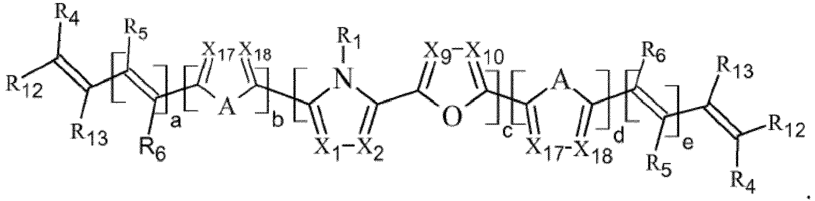

25. Composés selon la revendication 24, dans lesquels, indépendamment les uns des autres, A = O, S.

26. Composés selon l'une quelconque des revendications 24 ou 25, dans lesquels, indépendamment les uns des autres, X17, X18, X1, X2, X9 et X10 = C-R, les R étant choisis indépendamment les uns des autres dans un groupe constitué par H, halogène, alkyle en C1-C20 ramifié ou linéaire, cyclique ou à chaîne ouverte, R5 et R6 étant chacun indépendamment l'un de l'autre choisis parmi H, CN, F, aryle, hétéroaryle, alcényle en C2-C20, alcynyle, alkyle en C1-C20 ramifié ou linéaire, cyclique ou à chaîne ouverte, des atomes d'hydrogène de l'alkyle en C1-C20 pouvant être substitués, si les substituants R5 et R6 sont présents, une fermeture de cycle pouvant être formée entre R5 et R13 ou entre R6 et R13.

27. Composés selon l'une quelconque des revendications 24 à 26 précédentes, dans lesquels c = 1.

28. Composés selon l'une quelconque des revendications 24 à 27 précédentes, dans lesquels b = 1.

29. Composés selon l'une quelconque des revendications 24 à 28 précédentes, dans lesquels e = 1.

30. Composés selon l'une quelconque des revendications 24 à 29 précédentes, dans lesquels R4 et R12 sont chacun indépendamment l'un de l'autre choisis parmi H, CN, à condition que R4 et R12 ne représentent pas tous les deux H.

31. Utilisation d'un ou de plusieurs composés selon les revendications 1 à 30 dans un composant électronique organique.

32. Utilisation selon la revendication 31 dans un élément photoactif organique, de préférence dans une cellule solaire.

33. Dispositif électronique organique comprenant un ou plusieurs composés selon l'une quelconque des revendications 1 à 30.

34. Dispositif électronique organique selon la revendication précédente, comprenant une électrode et une contre-électrode et au moins une couche photoactive organique entre l'électrode et la contre-électrode, la couche photoactive organique comprenant au moins un des composés selon l'une quelconque des revendications 1 à 30.
